# EUROPEAN PATENT SPECIFICATION

(11) **EP 4 502 572 B1**
(45) Date of publication and mention of the grant of the patent: **02.12.2026**
(21) Application number: 23775077.3
(22) Date of filing: 24.03.2023
(51) Int. Cl.: G01N 11/04, G01N 33/49

(54) **METHOD AND SYSTEM FOR MEASURING A FLOW PROPERTY OF A LIQUID**
VERFAHREN UND SYSTEM ZUR MESSUNG EINER FLUSSEIGENSCHAFT EINER FLÜSSIGKEIT
PROCÉDÉ ET SYSTÈME DE MESURE D'UNE PROPRIÉTÉ D'ÉCOULEMENT D'UN LIQUIDE

(30) Priority: 25.03.2022 JP 2022050143
(43) Date of publication of application: 05.02.2025
(73) Proprietor: UNIVERSITY OF OCCUPATIONAL AND ENVIRONMENTAL HEALTH, JAPAN, Kitakyushu-shi, Fukuoka 8078555 (JP); KYUSHU INSTITUTE OF TECHNOLOGY, Kitakyushu-shi, Fukuoka 8048550 (JP)
(72) Inventor: OHNO, Koki, Kitakyushu-shi, Fukuoka 8078555 (JP); SATO, Hiroaki, Kitakyushu-shi, Fukuoka 8078555 (JP); UMEHARA, Takahiro, Kitakyushu-shi, Fukuoka 8078555 (JP); TSUKADA, Junichi, Kitakyushu-shi, Fukuoka 8078555 (JP); SAKAMOTO, Kenji, Iizuka-shi, Fukuoka 8208502 (JP)
(74) Representative: Winter, Brandl - Partnerschaft mbB
(86) International application number: PCT/JP2023/011748
(87) International publication number: WO 2023/182484

(56) References cited:
- WO-A1-2017/044055
- JP-A- 2006 145 351
- JP-A- 2021 001 762
- JP-A- 2021 001 762
- JP-A- H05 306 986
- JP-B2- 7 371 850
- KR-B1- 101 308 655
- KR-B1- 101 308 655
- US-A1- 2003 064 505
- US-A1- 2006 065 044

## Description

### Technical Field

The present invention relates to a method for measuring a flow property of a liquid and a measuring instrument.

### Background Art

Various experiments and the like using living body samples, bio samples, and the like are required to perform, for example, precise measurement and comparison of, for example, the viscosity of a small amount of liquid specimen, and measurement of the viscosity of blood coagulation or the like, which varies in a short time, in some cases.

Japanese Patent Laid-Open No. 2020-008342 discloses a body fluid viscosity measuring device that measures the viscosity of a body fluid, the body fluid viscosity measuring device including a flow path through which the body fluid flows with effects of force by capillary action and a calculation means that derives the viscosity of the body fluid by performing regression analysis based on a travel distance by which the body fluid has moved along the flow path and a movement time taken for the movement by the travel distance.

Japanese Patent Laid-Open No. 2021-001762 discloses a liquid collecting device including a body part and a switching valve, the body part including a minute flow path that has a capillary tube shape and includes a first opening part and a second opening part, the switching valve being connected to the first opening part side, and the switching valve includes a flow path connected to the body part, and a flow path opened to atmosphere and/or a flow path connected to a sealed container.

In addition, Kenji Sakamoto et al 2020 Jpn. J. Appl. Phys. 59 107002 relates to evaluation of viscosity and surface tension of low-volume samples using glass capillary and discloses that the viscosity coefficient of a sucrose solution of various concentrations is measured by methods related to Japanese Patent Laid-Open No. 2020-008342and Japanese Patent Laid-Open No. 2021-001762 and the like, and numerical values that match literature values are obtained.

US2003/064505 A1 describes a device and method for investigating the flowability of a physiological fluid sample.

WO2017/044055 A1 discloses a blood coagulation time measurement method and apparatus.

KR 101308655 B1 describes a viscosity measurement method using a disposable viscosity measurement apparatus.

### Summary of Invention

### Technical Problem

The viscosity and surface tension of a small amount of specimen (sample) can be measured by methods described in Japanese Patent Laid-Open No. 2020-008342 and Japanese Patent Laid-Open No. 2021-001762 and Kenji Sakamoto et al 2020 Jpn. J. Appl. Phys. 59 107002.

The inventors thought that the configurations of Japanese Patent Laid-Open No. 2020-008342 and Japanese Patent Laid-Open No. 2021-001762 and Kenji Sakamoto et al 2020 Jpn. J. Appl. Phys. 59 107002 have the potential for further applications. Then, the inventors discussed configurations for further utilization. Under such circumstances, it is an objective of the present invention to provide a measuring instrument, method, and the like that are capable of measuring a flow property such as the viscosity of a small amount of specimen.

### Solution to Problem

As a result of diligent research to solve the above-described problem, the present inventors discovered that the following invention meets the above-described objective, leading to the present invention.

Specifically, the present invention is defined by the method of claim 1 and the system of claim 9, preferred embodiments of both the method and the system are defined in the dependent claims.

### Advantageous Effect of Invention

According to the present invention, it is possible to measure the flow property of a small amount of specimen.

### Brief Description of Drawings

[Figure 1] Figure 1 is an overview diagram of a measuring instrument according to an embodiment of the present invention.
[Figure 2] Figure 2 is an overview diagram for description of the principle of a first embodiment according to the present invention.
[Figure 3] Figure 3 is an overview diagram of flow in a capillary tube for description of the principle of the first embodiment according to the present invention.
[Figure 4] Figure 4 is an overview diagram for description of the principle of a pressure adjustment unit that can be used for the present invention.
[Figure 5] Figure 5 is an overview diagram related to a pressure difference and a flow property for description of a principle according to the first embodiment of the present invention.
[Figure 6] Figure 6 is an overview diagram related to liquid level position detection with a sensor by a measuring instrument of the present invention.
[Figure 7] Figure 7 is a flowchart according to the first embodiment of the present invention.
[Figure 8] Figure 8 is an overview diagram of a capillary tube according to a second embodiment of the present invention.
[Figure 9] Figure 9 is a flowchart according to the second embodiment of the present invention.
[Figure 10] Figure 10 is an overview diagram related to liquid level position detection with a sensor according to the second embodiment of the measuring instrument of the present invention.
[Figure 11] Figure 11 is an overview diagram related to liquid level position detection with a sensor according to the second embodiment of the measuring instrument of the present invention.
[Figure 12] Figure 12 is an overview diagram related to disposition and the like of a capillary tube according to a fifth embodiment of the measuring instrument of the present invention.
[Figure 13] Figure 13 is an overview diagram related to a pressure difference of the capillary tube and a flow property according to the fifth embodiment of the present invention.
[Figure 14] Figure 14 is an overview diagram related to disposition and the like of a capillary tube according to a sixth embodiment of the measuring instrument of the present invention.
[Figure 15] Figure 15 is a graph of measurement results in an example of the present invention.
[Figure 16] Figure 16 is a graph of measurement results in the example of the present invention.
[Figure 17] Figure 17 is a graph of measurement results in the example of the present invention.
[Figure 18] Figure 18 is a graph of measurement results in the example of the present invention.
[Figure 19] Figure 19 is a graph of measurement results in the example of the present invention.
[Figure 20] Figure 20 is a graph of measurement results in the example of the present invention.
[Figure 21] Figure 21 is a graph of measurement results in the example of the present invention.
[Figure 22] Figure 22 is a graph of measurement results in the example of the present invention.

### Description of Embodiments

Embodiments of the present invention will be described below in detail, but the following description of components is an example (representative example) of aspects of the present invention. Note that in a case where an expression with "to" is used in the present specification, the expression is used as an expression including numerical values before and after "to".

### [Measuring instrument of the present invention] (not covered by the present invention)

A measuring instrument of the present invention is a measuring instrument configured to measure a flow property of a test liquid, the measuring instrument including: a push-back means configured to push back the test liquid, the test liquid having flowed into a capillary tube including a first opening part and a second opening part from the second opening part side toward the first opening part side, to the second opening part side; an adjustment valve for adjusting liquid flow in the capillary tube; and a measurement unit configured to measure an arrival time tn at a liquid level position Ln in the capillary tube.

The measuring instrument of the present invention may be, for example, a measuring instrument configured to measure a flow property of a test liquid, the measuring instrument including: a capillary tube including a first opening part and a second opening part; a pressure adjustment unit that is connected to the first opening part side and can be set to pressure with which the test liquid having flowed into the capillary tube is pushed back to the second opening part side; a switching valve for switching connection between the pressure adjustment unit and the capillary tube; a measurement unit configured to measure an arrival time tn at a liquid level position Ln in the capillary tube; and a push-back detection unit configured to detect that the test liquid in the capillary tube has been pushed back to the second opening part side and passed through.

### [Measuring method of the present invention] (not covered by the present invention)

A measuring method of the present invention is a method for measuring a flow property of a test liquid by using a measuring instrument including: a push-back means configured to push back the test liquid, the test liquid having flowed into a capillary tube including a first opening part and a second opening part from the second opening part side toward the first opening part side, to the second opening part side; an adjustment valve for adjusting liquid flow in the capillary tube; and a measurement unit configured to measure an arrival time tn at a liquid level position Ln in the capillary tube.

The measuring method of the present invention may be, for example, a method for measuring a flow property of a test liquid by using a measuring instrument including: a capillary tube including a first opening part and a second opening part; a pressure adjustment unit that is connected to the first opening part side and can be set to pressure with which the test liquid having flowed into the capillary tube is pushed back to the second opening part side; a switching valve for switching connection between the pressure adjustment unit and the capillary tube; a measurement unit configured to measure an arrival time tn at a liquid level position Ln in the capillary tube; and a push-back detection unit configured to detect that the test liquid in the capillary tube has been pushed back to the second opening part side and passed through.

Note that, in the present application, the measuring method of the present invention may be performed by the measuring instrument of the present invention, and their corresponding components may be mutually used.

The present invention has a configuration where a capillary tube can be repeatedly used. Accordingly, there is no influence of change of the capillary tube itself due to exchange of capillary tubes. Thus, a flow property such as minute viscosity change can be accurately detected.

A specimen that is an examination liquid having flowed through a capillary tube is pushed back to a flow start position by applying pressure and caused to flow into the capillary tube again to perform flow measurement. Thus, the same capillary tube is repeatedly used, and accordingly, reproducibility of measurement results is excellent. Moreover, a liquid reservoir part can be used as a reaction tank, and its displacement can be detected. Moreover, for example, temporal change of viscosity or the like that changes in a short time with a half-life of approximately one minute can be traced.

With conventional methods, there has been a probability of influence of the capillary tube itself, and it has been difficult to trace temporal change that occurs in a short time.

In the present invention, for example, the tilt of the capillary tube may be adjusted and measurement may be performed in a tilted state, and push-back of a test liquid in the capillary tube may be performed by adjusting pressure in the capillary tube or may be adjusted by applying another force. For example, influence of gravitational force or the like increases with the tilting, but push-back may be performed by using a tilted stage or the like that can change the tilt and orientation of the capillary tube.

### [Measuring instrument]

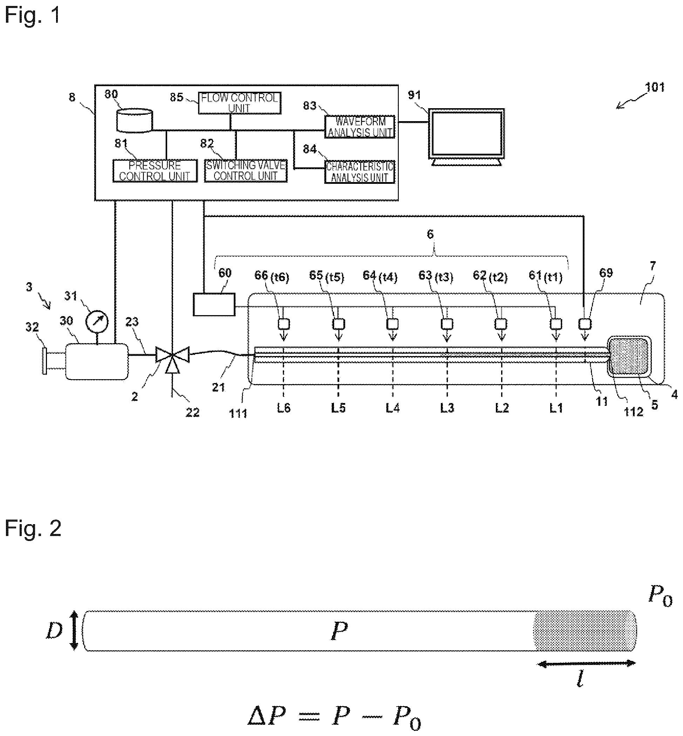

Figure 1 is a typical overview diagram of a measuring instrument according to an embodiment of the present invention. A measuring instrument 101 uses a capillary tube 11. The measuring instrument 101 includes the capillary tube 11, a switching valve 2, a pressure adjustment unit 3, a detection unit 6, and a control unit 8. A placement table 7 includes a liquid reservoir part 4 and can be disposed such that a second opening part 112 of the capillary tube 11 contacts a test liquid 5 housed in the liquid reservoir part 4.

### [Capillary tube 11]

The capillary tube 11 includes a minute flow path in a hollow capillary tube shape and includes a first opening part 111 and the second opening part 112 at respective ends of the minute flow path. The first opening part 111 is connected to the switching valve 2 through a flow path 21. The capillary tube 11 may be, for example, a capillary tube commercially available for applications such as sample spotting in chromatography and electrodes for electrophysiological experiments. The capillary tube includes those that exhibit capillary action, such as hydrophilic microchannels.

The capillary tube 11 is a flow path including a minute flow path in a capillary tube shape and having a size with which capillary action occurs. The amount and flow of liquid entering the minute flow path are determined by surface tension, capillary tube force determined by the angle of contact with the flow path, the size of the flow path, and the like, pressure in a minute flow path space, frictional force, and gravitational force. The radius of a minute flow path suitable for viscosity measurement that utilizes such capillary action is approximately 1.0 mm or smaller or is approximately 0.5 mm or smaller.

The present invention is suitable for use with a small amount of liquid. For example, the present invention is suitable for measurement of blood, saliva, or the like, and these contain water as a main medium. In a case where those that contain water are measured, the capillary tube 11 is preferably one that is hydrophilic so that capillary action is likely to occur. Furthermore, a hydrophobic capillary tube or the like may be used for those that contain a large amount of oil components. The capillary tube 11 may be, for example, those made of glass.

### [Switching valve 2]

The switching valve 2 is connected to the first opening part 111 through the flow path 21. Furthermore, the switching valve 2 can switch opening and closing to atmosphere, and connection to the pressure adjustment unit 3. For this switching, a flow path 22 and a flow path 23 are used as appropriate. Since capillary action relatively immediately proceeds, the switching valve 2 is preferably an electromagnetic valve that can instantaneously control measurement start and measurement stop with electric signals.

The switching valve 2 coordinates the timing of flow and the timing of measurement in a coupled manner with a sensor configured to switch stop/start of flow of a liquid that is housed in the liquid reservoir part and infiltrates the capillary tube from the second opening part 112.

In the measuring instrument 101, the switching valve 2 is preferably an electromagnetic valve. Furthermore, it is preferable that a stop sensor that detects passing through a liquid level be provided on the near side of the first opening part 111 of the capillary tube 11, a signal that closes the switching valve 2 be transmitted when the stop sensor has detected a liquid level, and accordingly, the switching valve 2 be closed to stop liquid flow in the capillary tube 11. Alternatively, detection by a sensor attached for timing, such as a sensor 66 may be also used for stopping. The capillary tube 11 may discharge a housed liquid and may be cleaned and rinsed and repeatedly used, as appropriate.

Furthermore, instead of using a signal that stops flow when the stop sensor has detected a liquid level, a signal that stops flow may be output when a certain time has elapsed after flow start. The certain time until the stop may be set as appropriate in accordance with the length of the capillary tube, the flow speed of a liquid, and the like. The upper limit of the time until the stop may be set, such as 10 seconds, 5 seconds, 3 seconds, or 2 seconds.

### [Flow path 21]

The first opening part 111 of the capillary tube 11 and the switching valve 2 may be connected by an optional means. The capillary tube 11 may be connected by using, for example, a flexible tube as the flow path 21 so that replacement can be easily performed. The flow path 21 is preferably one that is hydrophobic. As partially described above, the capillary tube 11 is preferably one that is hydrophilic so that capillary action is likely to occur. Meanwhile, it is preferable that an examination liquid do not excessively enter to the switching valve 2 side beyond the first opening part 111 of the minute flow path. Thus, a hydrophobic tube is used as the flow path 21 so that capillary action is unlikely to occur in the flow path 21 and an examination liquid stops near the first opening part 111.

### [Push-back means]

The measuring instrument may include a push-back means. In the measuring instrument 101 in Figure 1, the test liquid 5 first flows into the capillary tube 11 from the second opening part 112 side toward the first opening part 111 side of the capillary tube 11. The push-back means is used to push back the test liquid 5 from a state of having flowed to a predetermined position to the second opening part 112 side for remeasurement, exchange of test liquids, or the like. The push-back means of the measuring instrument 101 has a configuration using the pressure adjustment unit 3 and the like.

### [Adjustment valve]

The measuring instrument includes an adjustment valve for adjusting liquid flow in the capillary tube. The adjustment valve is a valve used to change flow speed or the like by, for example, stopping or pushing back liquid flow in the capillary tube or changing pressure. In the measuring instrument 101, for example, a switching valve 22 for changing, for example, the state of pressure on the first opening part 111 side or the like when, for example, causing the test liquid 5 to flow into the capillary tube 11, pushing back the test liquid 5 by pressure, or stopping flow of the test liquid 5 functions as the adjustment valve.

### [Pressure adjustment unit 3]

The pressure adjustment unit 3 is connected through the switching valve 2 to allow connection with the first opening part 111 side. The pressure adjustment unit 3 may be held at the atmospheric pressure except for during measurement of a flow time. The pressure adjustment unit 3 may be adjusted to pressure other than the atmospheric pressure at measurement of a flow time and used. The pressure adjustment unit 3 may be one that allows understanding that the pressure adjustment unit 3 is adjusted to a plurality of pressures. The pressure adjustment unit 3 is a part that is adjusted to a negative pressure, a positive pressure, or the atmospheric pressure. The pressure adjustment unit 3 can be set to at least pressure with which a liquid having flowed into the capillary tube 11 is pushed back to the second opening part 112 side.

### [Pressure adjustment using air reservoir container and adjustment piston]

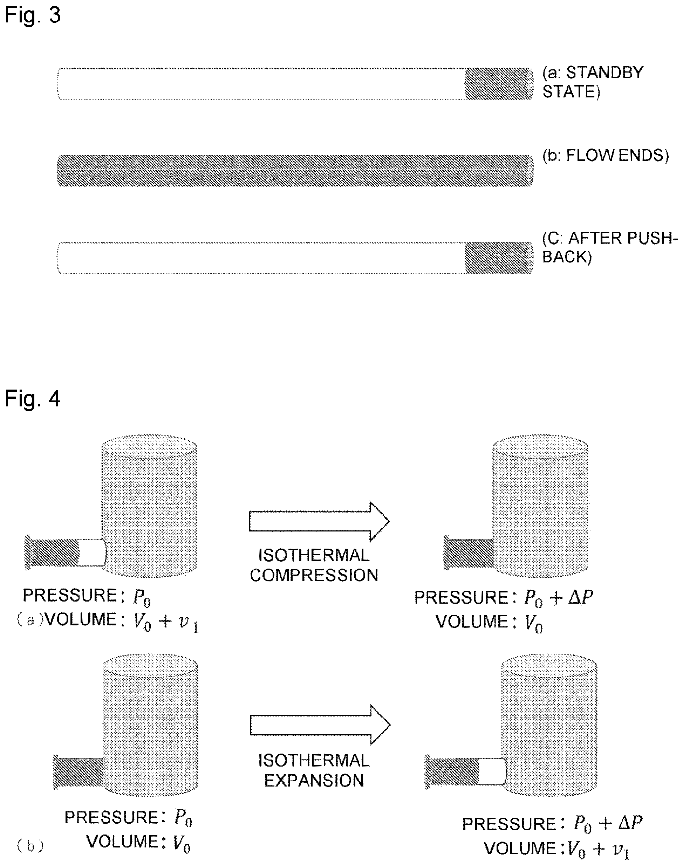

The pressure adjustment unit 3 preferably includes a container 30 as an air reservoir and an adjustment piston 32 connected to the air reservoir and exhibits isothermal compression or isothermal expansion by changing the total volume of an inside connected to the air reservoir by the adjustment piston. The procedure of pressure adjustment using the air reservoir and the piston will be described below (Figure 4(a) and Figure 4(b)). By performing such pressure adjustment, it is possible to perform minute pressure adjustment in the capillary tube, which is required for the present invention, without performing complicated control without using a pump, a barometer, or the like.

### [Combination of pressure adjustment unit 3 and switching valve 2]

States (1) to (4) below can be achieved with combination of the pressure adjustment unit 3 and the switching valve 2 (hereinafter simply referred to as combination).
(1) The pressure adjustment unit 3 is isolated from atmosphere. The flow path 21 is closed.
(2) The pressure adjustment unit 3 is opened to atmosphere. The flow path 21 is closed.
(3) The pressure adjustment unit 3 is isolated from atmosphere. The pressure adjustment unit 3 and the flow path 21 are connected.
(4) The pressure adjustment unit 3 is opened to atmosphere. The pressure adjustment unit 3 and the flow path 21 are connected.

### [Administration of specimen]

A specimen as an examination liquid is administered to the second opening part in a state (combination (1) or (2)) in which the flow path 21 is closed. The specimen is sucked into the capillary tube by surface tension. Air pressure inside the capillary tube rises, balances with the surface tension, and stops. The specimen that is not sucked remains in a liquid reservoir. In a case where the specimen is in an extremely small amount (up to 5 uL), all administered specimen is sucked into the capillary tube and stays near the second opening part 112.

### [Pressure setting 1; flow under atmospheric pressure]

Specimen administration is performed in the state of combination (2). The following measurement is possible when a sufficient amount of the specimen remains in the liquid reservoir. After the administration, transition is made to the combination state (4) by an operation of the switching valve. The specimen is driven by surface tension, flows in the capillary tube, and arrives at the first opening part 111 of the capillary tube 11. The switching valve 2 is actuated and the flow path 21 is closed, and accordingly, transition is made to the combination state (2). Flow stops.

### [Pressure setting 2; positive pressure]

Specimen administration is performed in the state of combination (2). The internal pressure of the pressure adjustment unit is equal to the atmospheric pressure. Transition is made to combination (1) in a state in which the adjustment piston is pulled out as illustrated on the left side in Figure 4(a). The pressure adjustment unit is isolated from atmosphere. Thereafter, the adjustment piston is pushed in. The internal pressure of the pressure adjustment unit increases by ΔP of the following expression. Here, P0 is the atmospheric pressure. V0 is the volume of the container. v1 is the volume in the piston. $ΔP = P 0 ⋅ v 1 / V 0$

The switching valve is operated in this state, and transition is made to combination (3). Flow recording under the positive pressure is achieved. Simultaneously with flow end, the switching valve is operated to return to combination (2). The pressure adjustment unit 3 is opened to the atmospheric pressure, and the flow path 21 is closed.

### [Pressure setting 3; negative pressure]

Specimen administration is performed in the state of combination (2). The internal pressure of the pressure adjustment unit is equal to the atmospheric pressure. Transition is made to combination (1) in a state in which the adjustment piston is pushed in as illustrated on the left side in Figure 4(b). The pressure adjustment unit 3 is isolated from atmosphere. Thereafter, the adjustment piston is pulled out. The internal pressure of the pressure adjustment unit changes by ΔP of the following expression (note that ΔP is negative, and accordingly, the inside of the pressure adjustment unit becomes a negative pressure). Here, P0 is the atmospheric pressure. V0 is the volume of the container. v1 is the volume in the piston. $ΔP = - P 0 ⋅ v 1 / V 0$

The switching valve 2 is operated in this state, and transition is made to combination (3). Flow recording under the negative pressure is achieved. Simultaneously with flow end, the switching valve 2 is operated to return to combination (2). The pressure adjustment unit 3 is opened to the atmospheric pressure, and the flow path 21 is closed.

### [Pressure meter 31]

A pressure meter 31 is preferably attached to the pressure adjustment unit 3 to measure its pressure. The pressure meter 31 may be various pressure meters that measure the atmospheric pressure. In the present invention, in particular, it is preferable to use a differential pressure meter that can measure pressure with scales of approximately 10 Pa steps, 1 Pa steps, or 0.5 Pa steps in the measurement range of approximately ±10,000 Pa, ±5,000 Pa, ±1,000 Pa, ±500 Pa, or ±300 Pa with reference to the atmospheric pressure.

The present invention uses the negative pressure as pressure and thus is also suitable for measurement of a specimen with high viscosity. The specimen with high viscosity has a low flow property due to capillary action and thus is difficult to measure with the atmospheric pressure, the positive pressure, and the like in some cases, but use of the negative pressure improves the flow property and makes measurement easy. In a case where the negative pressure is used to measure the specimen with high viscosity, the negative pressure may be 5,000 to 10,000 Pa.

The pressure adjustment unit 3 may be, for example, one that can connect a pressure container and a vacuum pump through a valve and prepare pressure in accordance with the degree of opening and closing of the valve. The pressure adjustment unit 3 only needs to be able to determine and manage pressure, and as described above, may be attached with the pressure meter 31 to measure pressure at that time and perform correlation calculation and the like by using the measured pressure. Furthermore, pressure when conditions related to the pressure container of the pressure adjustment unit 3 and the like are set may be determined and may be regarded and used as the pressure. Specifically, for example, a syringe may be attached to the container so that pressure can be determined from a suction scale of the syringe.

### [Liquid reservoir part 4]

The liquid reservoir part 4 houses a specimen as a test liquid. Furthermore, the second opening part 112 of the capillary tube 11 contacts the test liquid housed in the liquid reservoir part 4.

The amount of the test liquid housed in the liquid reservoir part 4 may be set as appropriate based on the kind, various conditions (size of the capillary tube) that affect the viscosity coefficient and surface tension, and the like, and may be in ranges such as 500 µL or smaller, 300 µL or smaller, 100 µL or smaller, and 50 µL or smaller, and its lower limit may be a small amount such as 1 µL or larger, 5 µL or larger, and 10 µL or larger.

### [Test liquid 5]

The test liquid 5 may use, as an examination liquid, any liquid with which capillary action occurs. The liquid may be one in which various kinds of components are dissolved or dispersed. A living-body sample such as blood or saliva may be targeted. Furthermore, emulsion, polymer solution, particle suspension, and the like may be used. With the measuring instrument 101, the flow property of the test liquid 5 in the capillary tube 11 can be measured. Furthermore, the capillary tube 11, and the flow path 21 as necessary, can be removed and easily discarded.

### [Measurement unit 6]

A measurement unit 6 measures the arrival time tn at the liquid level position Ln in the capillary tube. The liquid level position Ln is a detection target position at a predetermined distance from the second opening part 112, and the arrival time tn is an arrival time from the second opening part 112 to the liquid level position Ln. The measurement unit 6 includes sensors 61 to 66 and a timing unit 60. The sensors 61 to 66 are sensors that detect passing of the liquid level of an examination liquid flowing in the capillary tube 11. The sensors 61 to 66 are those for detecting flow time and passing speed at the flow distance of a liquid flowing inside the capillary tube 11. The sensors 61 to 66 are arranged alongside in the minute flow path of the capillary tube 11 and provided in plurality (in Figure 1, six) at a predetermined interval. Passing of the liquid level in the minute flow path can be detected at positions to which the sensors 61 to 66 correspond. The sensors 61 to 66 are arrayed on the placement table 7.

For example, the sensors 61 to 66 may be each an optical sensor that detects passing of the liquid level in the minute flow path. When a liquid varies in the minute flow path, its liquid level becomes a state that is significantly easy to be observed by an optical means due to refractive index difference and/or light scattering difference between the liquid and gas in a space. Passing of the liquid level can be detected by the sensors 61 to 66 at the corresponding positions.

Sensors used in the measuring instrument according to the present invention include one that detects passing of the liquid level by a reflective method, one that performs detection by a transmissive method, and one that acquires information of a flow state as a moving image or the like by using a line sensor or an area sensor and detects flow time and passing speed based on the moving image or the like.

The measuring instrument 101 detects flow time at the flow distance of a liquid and passing speed at the flow distance by using the sensors 61 to 66. For this, the measuring instrument 101 includes the timing unit 60 that acquires, for example, times at which passing of the liquid level is detected by the sensors 61 to 66.

In the present invention, the flow property may be measured based on flow time or the like corresponding to, for example, the inflow direction from the second opening part 112 to the first opening part 111 and the pressure adjustment unit 3 side, or the flow property may be measured based on flow time or the like corresponding to, for example, the push-back direction from the first opening part 111 and the pressure adjustment unit 3 side to the second opening part 112 side due to push-back. The arrival time tn at the liquid level position Ln in the capillary tube can be obtained by selecting and using suitable sensors in accordance with direction at that time.

### [Timing unit 60]

The timing unit 60 measures time (flow time) at which the liquid level passes through positions corresponding to the sensors 61 to 66, and speed (passing speed) when the liquid level passes by their flow distances. The timing unit 60 is connected to the switching valve 2 as well and measures detection times at the sensors 61 to 66 upon switching of the switching valve 2.

A measurement unit that measures a flow time tn (t1 to t6) until the liquid level of an examination liquid 5 passes and a passing speed vn (v1 to v6) at a flow distance Ln (L1 to L6) in the capillary tube 11 is constituted by the sensors 61 to 66 and the timing unit 60.

In the measuring instrument 101, the switching valve 2 is preferably an electromagnetic valve. Furthermore, it is preferable that a stop sensor that detects the liquid level position in the capillary tube 11 be provided, a signal that stops the switching valve 2 be transmitted when the stop sensor has detected the liquid level, and accordingly, the switching valve 2 be closed to stop so that liquid flow is stopped in the capillary tube 11. The stop sensor may be attached for stopping near the first opening part 111 of the capillary tube 11 for stopping. Alternatively, detection by a sensor attached for timing, such as the sensor 65 or the sensor 66 may be used for stopping. In a case where a liquid enters the flow path 21, the measuring instrument 101 is contaminated and recovery work is cumbersome in some cases.

### [Push-back detection unit 69]

A push-back detection unit 69 detects that a liquid in the capillary tube 11 has been pushed back to the second opening part 112 side and passed through. The push-back detection unit 69 may be, for example, a sensor that detects the liquid level similarly to the sensors 61 to 66. Furthermore, any of the sensors 61 to 66, for example, the sensor 61 may be a sensor for use together with the push-back detection unit 69. The push-back detection unit 69 preferably detects a position in the capillary tube 11 to stop the leading end of the liquid level in the capillary tube 11 so that gas does not enter the liquid reservoir part 4 through the capillary tube 11.

### [Placement table 7]

The placement table 7 supports the capillary tube 11. A substrate on which the capillary tube 11, the liquid reservoir part 4, and the sensors 61 to 66 are arrayed is provided on the placement table 7. Furthermore, the placement table 7 is configured so that the minute flow path of the capillary tube 11 has a predetermined tilt. Furthermore, the placement table 7 is configured so that the second opening part 112 of the capillary tube 11 contacts the test liquid 5 in a state in which the test liquid 5 is housed in the liquid reservoir part 4.

### [Control unit 8]

The control unit 8 is a part that performs control and processing of various kinds of functions of the measuring instrument. The control unit 8 may be one that includes a storage unit 80, a pressure control unit 81, a switching valve control unit 82, a waveform analysis unit 83, a characteristic analysis unit 84, a flow control unit 85, and the like.

### [Storage unit 80]

The storage unit 80 is a part that stores measurement values obtained when measurement of the present invention is performed, formulae for calculating processing data, programs for performing the processing, and the like.

The pressure control unit 81, for example, sets the pressure of the pressure adjustment unit 3 in accordance with a measurement situation. In the present invention, the pressure adjustment unit 3 can operate while switching the pressure to the positive pressure, the negative pressure, the atmospheric pressure, and the like as appropriate. Adjustment of the adjustment piston, cooperation with the switching valve (electromagnetic valve), management of the measured value of a pressure meter, and the like for this are performed.

The switching valve control unit 82 can switch the switching valve 2 as appropriate. In accordance with a purpose such as a measurement state based on a flow situation in the capillary tube 11, and a flow direction, the switching valve 2 connects the capillary tube 11 to the pressure adjustment unit 3, opens the capillary tube 11 to the atmospheric pressure, or sets the capillary tube 11 to a closed state without connection to any of them, for example.

The waveform analysis unit 83 performs analysis of passing time, a passing position, a flow direction, and the like by analyzing a waveform such as a peak measured by the measurement unit 6. The characteristic analysis unit 84 analyzes flow properties of an examination liquid, such as viscosity and surface tension, based on the contents of analysis of a waveform due to a liquid level change along with flow. The flow control unit 85 controls motion of the entire measuring instrument in accordance with each flow in accordance with a measurement purpose.

### [Display unit 91]

A display unit 91 is a monitor that displays measured flow properties, various conditions, regression equations of calculation processes, and the like.

The measuring instrument 101 can be implemented by applying a program for functioning as each component of the control unit 8 or the like to a personal computer, a tablet terminal, a smartphone, or the like.

### [Amount of liquid that flows in (enters) capillary tube]

Figure 2 is an overview diagram for description of the principle of a first embodiment according to the present invention. A capillary tube with an inner diameter D is horizontally placed. One end (right end of the diagram) of the capillary tube is in contact with the liquid reservoir (omitted). The liquid reservoir is held at an atmospheric pressure P0, and the inside of the capillary tube is held at pressure P. A specimen as an examination liquid in the liquid reservoir is driven by resultant force of surface tension and a pressure difference ΔP and enters and moves in the capillary tube. The entering distance of the specimen is represented by l. The case of ΔP > 0 is referred to as the positive pressure, and the case of ΔP < 0 is referred to as the negative pressure.

The viscosity coefficient of the specimen is represented by η, and the surface tension is represented by σ Elapsed time since flow start is represented by t. Temporal change of the entering distance l of the specimen is expressed by Expression (1).

The coefficient D/16η(4σ - DΔP) of t on the right hand side is referred to as a "flow coefficient" and abbreviated as IWs. IWs is expressed by Expression (2).
[Math. 1] ${l}^{2} = \frac{D}{16 η} \left(4σ-DΔP\right) t$ (1)
[Math. 2] $IWs = \frac{Dσ}{4 η} - \frac{{D}^{2}}{16 η} Δ P$ (2)

Symbols used in the above-described expressions and the present application are as follows.
D: capillary tube inner diameter (diameter) (mm)
σ: specimen surface tension (mN/m)
η: specimen viscosity coefficient (mPa·s)
l: length (mm) of an examination liquid having entered the tube
t: elapsed time (ms)
ΔP (Pa): difference ΔP = Pn - P0 between the container internal pressure (specified pressure Pn) and the atmospheric pressure (P0) at measurement

The measuring instrument of the present invention and the measuring method of the present invention evaluate the flow property of a test liquid from the liquid level position, the flow time, and the like based on such behavior of the liquid in the capillary tube. As for the flow property, relative measurement may be performed for a plurality of liquids or the same liquid by simply using time as an indicator, and viscosity or the like may be measured by using known values and the like as well.

### [Push-back of liquid in capillary tube]

Figure 3 is an overview diagram of flow in the capillary tube for description of a principle according to the first embodiment of the present invention. In the upper part, a is a standby state. In the middle part, b is when flow ends. In the lower part, c is after push-back.

From the standby state in Figure 3(a), the switching valve is opened in a state in which the container internal pressure is held at a value smaller than a critical pressure. The specimen is sucked into the capillary tube from the liquid reservoir. Motion of its leading end is expressed by Expression (1).

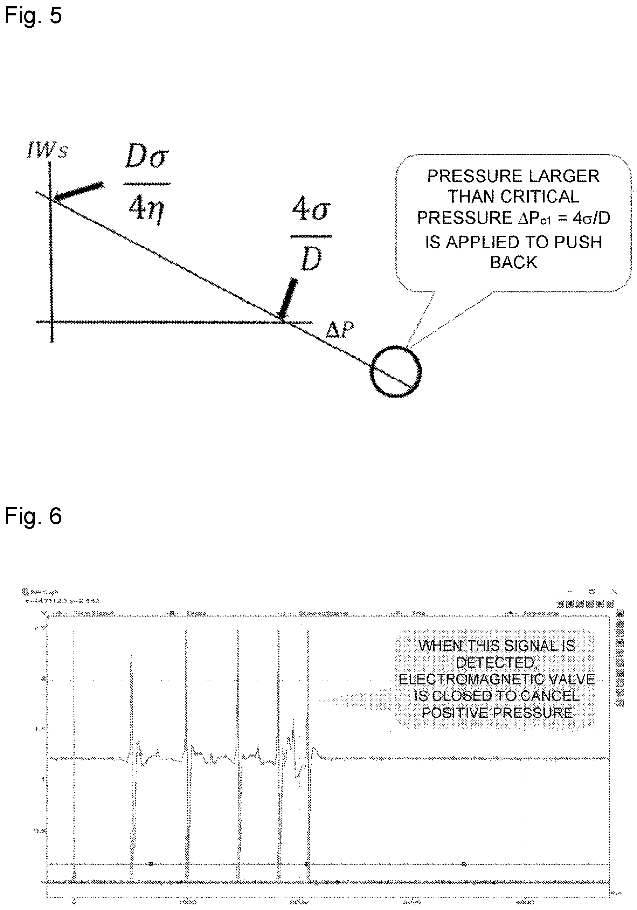

A signal example illustrated in Figure 6 to be described later is a signal of pure water when push-back is performed with the positive pressure larger than the critical pressure inside the container. The switching valve is closed when it is sensed that the specimen has arrived at a stop sensor. The capillary tube internal pressure immediately reaches the critical pressure, and motion of an examination liquid stops.

### [Adjustment of pressure adjustment unit]

Figure 4 is an overview diagram for description of the principle of the pressure adjustment unit that can be used for the present invention. The pressure adjustment unit can be set to the positive pressure by using isothermal compression as illustrated in Figure 4(a). In a case where isothermal compression is performed, the pressure inside the container before the compression is equal to the atmospheric pressure P0. When the pressure inside the container after the compression is represented by P0 + ΔP, an equation "P0(V0 + v1) = (P0 + ΔP)V0" holds. From this, "ΔP = P0.v1/V0" can be obtained. Furthermore, the negative pressure can be set through isothermal expansion by oppositely using the principle as illustrated in Figure 4(b).

Figure 5 is an overview diagram related to the pressure difference and the flow property for description of a principle according to the present invention.

When ΔP is zero, IWs described above is "Dσ/4η". When ΔP is "4σ/D", IWs is zero. In other words, liquid does not flow. The liquid can be pushed back by setting pressure with which IWs is a negative value. The value of the y-intercept "Dσ/4η" of this plot indicates motion under the atmospheric pressure. From this value, the ratio of the viscosity coefficient η and the surface tension σ of the test liquid can be determined. The value of the surface tension of the test liquid can be calculated from the x-intercept "4σ/D" of this plot. The value "ΔP_{c1} = 4σ/D" of the x-intercept is named as the critical pressure in the first embodiment of the present invention.

### [Critical pressure]

When the specimen remains in the liquid reservoir, the critical pressure or larger is needed for pushing back. The critical pressure is, for example, approximately 400 Pa for the inner diameter of 0.68 mm. This critical pressure value depends on the capillary tube inner diameter and the specimen surface tension. However, when the difference from the critical pressure is small, the speed of push-back is extremely slow, and thus the critical pressure + 100 Pa or larger is preferable. Typically, approximately 600 to 700 Pa may be pressure for pushing back. However, when this pressure is too large, "return" is too fast and potentially becomes turbulence and unstable.

Figure 6 is an overview diagram related to liquid level position detection with a sensor by the measuring instrument of the present invention. Figure 6 is obtained by detecting a signal when a liquid is pushed back. A large potential change occurs when the liquid level passes through the sensor. When a signal for the sensor selected as the push-back detection unit 69 is detected, the electromagnetic valve is closed to cancel the positive pressure. Accordingly, flow of the liquid in the capillary tube is stopped.

### [First Embodiment]

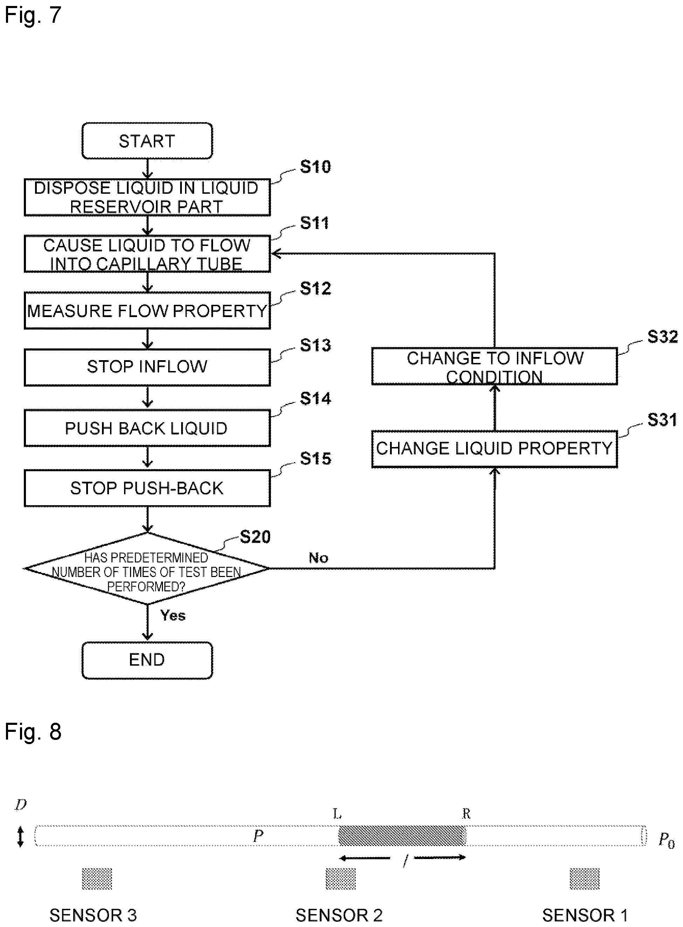

The first embodiment of the present invention uses push-back of a test liquid in a horizontally installed capillary tube by setting the pressure of the pressure adjustment unit to the positive pressure larger than the critical pressure ΔP_{c1} and stop of the push-back at a scheduled position. Figure 7 is a flowchart according to the first embodiment of the present invention.

Step S10 is a step of disposing the liquid in the liquid reservoir part.

Step S11 is a step of bringing the second opening part into contact with the liquid in the liquid reservoir part to cause the liquid to flow to the inside of the capillary tube from the second opening part.

Step S12 is a step of switching the switching valve to connect the first opening part of the capillary tube to a part that is set to a condition when the flow property is measured, thereby causing the liquid to flow in the capillary tube and measuring the flow property at inflow. For example, the first opening part may be opened to the atmospheric pressure for inflow or may be set to the negative pressure for inflow to more actively cause inflow.

Step S13 is a step of stopping inflow of the liquid by adjusting the switching valve on the first opening part side when stop position of inflow in the capillary tube is exceeded.

Step S14 is a step of pushing back the liquid by switching the switching valve on the first opening part side to connect the first opening part to the pressure adjustment unit being set to the critical pressure or larger.

Step S15 is a step of stopping by switching the switching valve when the liquid in the capillary tube has passed through the push-back detection unit.

Step S20 is a step of determining whether a predetermined number of times of a test has been performed. Transition is made to step S31 in a case where the predetermined number of times of the test has not yet completed (No).

Step S31 is a step of performing processing for performing the test with a changed test condition as appropriate, and the property of the liquid in the liquid reservoir part is changed through, for example, a temporal change, a temperature change, or reaction with a reagent or the like.

Step S32 is a step of changing the first opening part side to an inflow condition after a predetermined change has ended.

Thereafter, the flow property of the liquid in that state is measured again through steps S11 to S15.

Then, whether a test number of times of the test has been performed is checked and repeated at step S20 until the predetermined number of times of the test is performed. The test is completed in a case where the predetermined number of times of the test has been performed (Yes).

In this manner, the measuring method according to the first embodiment of the present invention can perform the test in a state in which the measuring instrument includes the liquid reservoir part that contacts the second opening part side. This measures the flow property when the liquid housed in the liquid reservoir part has flowed into the capillary tube from the second opening part.

The method of the present invention may include a first push-back process that the pressure adjustment unit is connected to the first opening part with the positive pressure relative to the critical pressure, the liquid having flowed into the capillary tube is sent from the second opening part to the liquid reservoir part, and push-back is stopped when the liquid is pushed back to the push-back detection unit.

Furthermore, the method of the present invention may include, after the push-back process, a displacement measurement process that the state of the liquid in the liquid reservoir part is changed and the flow property of the liquid in the capillary tube is measured.

The test in the first embodiment is a method by which repeated tests can be immediately performed. Thus, for example, slight displacement of the flow property of a test liquid can be immediately measured with a small amount of the test liquid. For example, influence of change in time, temperature, and the like can be measured. Furthermore, a test target component may be mixed in the liquid reservoir part to change the state of the liquid in the capillary tube, thereby measuring influence of its reaction.

### [Second Embodiment]

Figure 8 is an overview diagram of a capillary tube according to a second embodiment of the present invention. The second embodiment measures the flow property of a liquid housed inside the capillary tube, which is horizontally installed, in an amount less than the volume capacity of the capillary tube. Since the liquid is housed in the capillary tube, the left end (L) of the liquid and the right end (R) of the liquid exist. The flow property is measured when the liquid is moved in directions by adjusting the pressure difference between the first opening part side and the second opening part side.

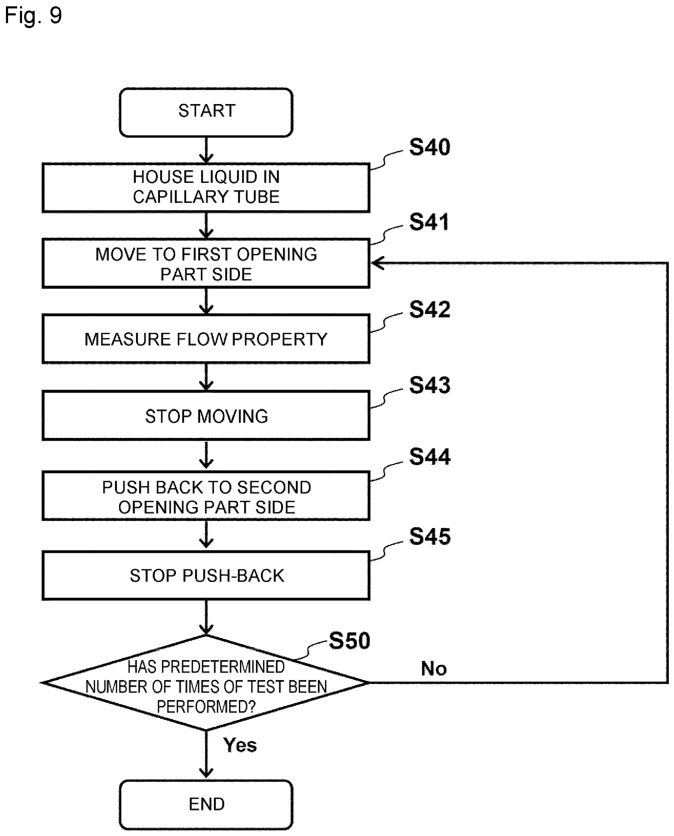

Figure 9 is a flowchart according to the second embodiment of the present invention.

Step S40 is a step of housing the liquid in the capillary tube. In the housing, only the liquid in an amount that can be housed in the capillary tube may be disposed and the total amount thereof may be housed in the liquid reservoir part, or the capillary tube may be made contact with the test liquid and then separated from the test liquid after housing, and thereafter, may partially house air to achieve a housed state. Inflow of the liquid from the second opening part is stopped when volume capacity for testing is housed.

Step S41 is a step of moving the liquid to the first opening part side by setting the pressure on the first opening part side to the negative pressure relative to the second opening part side and switching the first opening part side to the inflow condition.

Step S42 is a step of measuring the flow property of the flowing liquid.

Step S43 is a step of stopping inflow of the liquid by adjusting the switching valve on the first opening part side when an inflow stop position is exceeded.

Step S44 is a step of pushing back the liquid by connecting the first opening part to the pressure adjustment unit with the positive pressure relative to the second opening part side. The flow property at the push-back may be measured.

Step S45 is a step of stopping the push-back by switching the switching valve when the leading end of the liquid has passed through the push-back detection unit.

Step S50 is a step of checking whether a predetermined number of times of the test is performed after the push-back is stopped. Before completion (No), the process returns to step S41 again to perform the test. When the predetermined number of times of the test has ended (Yes), the process ends.

When a specimen as the test liquid is smaller than the volume of the capillary tube, all specimen administered to the liquid reservoir is sucked into the capillary tube and no specimen remains in the liquid reservoir. In this case, surface tension of the same size acts in opposite directions at the respective ends of the examination liquid in the capillary tube. Accordingly, no surface tension is involved in motion of a liquid column in the capillary tube.

The liquid column performs uniform motion when pressure is applied. The motion speed of the liquid column is proportional to the pressure difference and inversely proportional to the viscosity. The motion speed v is expressed by Expression (3). The viscosity of the specimen can be obtained by measuring the motion speed. When the examination liquid is all encapsulated in the capillary tube, the specimen can be reciprocated by setting the capillary tube internal pressure to be larger than the atmospheric pressure or smaller than the atmospheric pressure.
[Math. 3] $v = \frac{Δ {PD}^{2}}{32 ηl}$ (3)

Symbols used in Expression (3) are as follows.
v: flow speed (mm/ms) of the liquid column
D: inner diameter (mm) of the capillary tube
l: length (mm) of the examination liquid housed in the tube
η: viscosity coefficient (mPa·s) of the liquid specimen
ΔP: pressure difference (Pa) between the ends of the liquid column

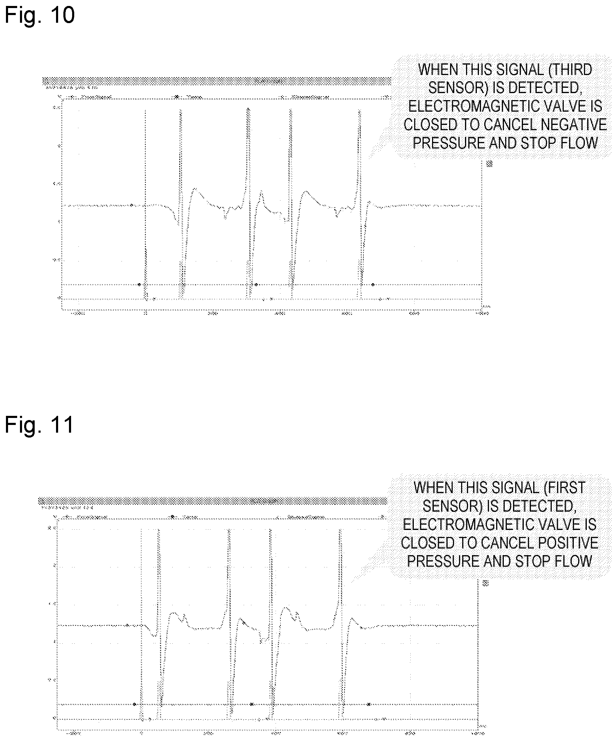

Figure 10 is an overview diagram related to liquid level position detection with a sensor by the measuring instrument of the present invention. Figure 10 is a signal of uniform motion in the left direction with the negative pressure (97 Pa).

Figure 11 is an overview diagram related to liquid level position detection with a sensor by the measuring instrument of the present invention. Figure 11 is a signal of uniform motion in the right direction with the positive pressure (97 Pa).

In tests of Figures 10 and 11, a capillary tube with the inner diameter D was horizontally placed and three optical sensors were disposed at equal intervals in the vicinity of the capillary tube. The volume of a specimen as an examination liquid was approximately 1/10 of the internal volume of the capillary tube. When the examination liquid is injected from the right end of the capillary tube in a state in which the inside of the capillary tube is held at the atmospheric pressure, the specimen is all sucked into the capillary tube by surface tension. Surface tensions that act at the L and R ends of the liquid column of the test liquid have the same value in opposite directions.

In a case of P = P0, the liquid column is at rest in the capillary tube. When the pressure adjustment unit is connected to the first opening part of the capillary tube on the left side to set the negative pressure (P < P0), the liquid column performs uniform motion in the left direction in the capillary tube. When the pressure adjustment unit is connected to the first opening part of the capillary tube on the left side to set the positive pressure (P > P0) in the capillary tube, the liquid column performs uniform motion in the right direction in the capillary tube.

In this manner, the second embodiment of the present invention includes a second push-back process that the pressure adjustment unit is connected to the first opening part with pressure with which a liquid housed in the capillary tube is pushed back to the second opening part side, the liquid housed in the capillary tube is pushed back to the second opening part side, and the push-back is stopped when the liquid is pushed back to the push-back detection unit. Accordingly, it is possible to measure stability when a plurality of times of measurement is performed with reciprocating motion and measure, for example, change of the flow property when temperature, time, pressure, and the like are changed.

### [Push-back pressure]

As in the second embodiment, when an extremely small amount of a specimen is a specimen liquid column in a horizontally installed capillary tube and the total amount thereof is in the capillary tube in the length of approximately 15 mm or less, the liquid column can be moved with pressure of approximately 100 Pa. The liquid column moves sufficiently fast even with such pressure. Note that there is no dependency on specimen surface tension.

### [Third Embodiment]

A third embodiment of the present invention may be such that each of a plurality of test liquids is measured by using the same capillary tube. The first opening part is connected to the pressure adjustment unit having been adjusted to pressure with which the liquid in the capillary tube is pushed back to the second opening part side, and the test liquid housed in the capillary tube is discharged from the capillary tube and used for the test of another test liquid.

### [Fourth Embodiment]

A fourth embodiment of the present invention may be such that a reagent to be mixed and reacted with a liquid is disposed inside the capillary tube. For example, the reagent is disposed near the sensor 66 in Figure 1 and the flow property before mixture with the reagent is measured at first passing through the capillary tube, and then, the liquid that is made contact with the reagent is pushed back and caused to flow again so that the flow property after mixture with the reagent can be measured.

### [Fifth Embodiment]

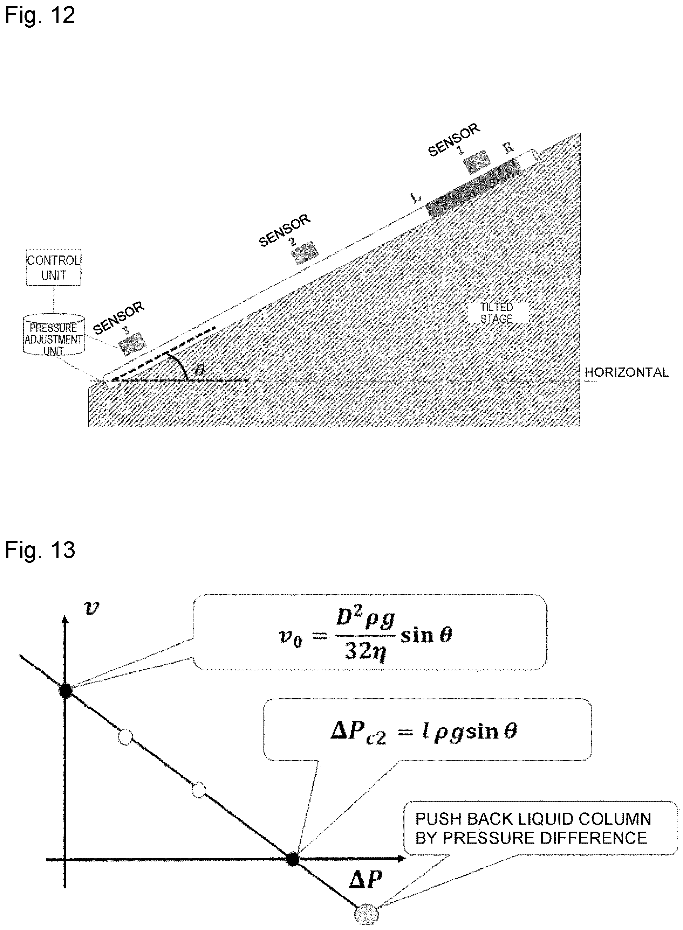

Figure 12 is an overview diagram related to disposition and the like of a capillary tube according to a fifth embodiment of the measuring instrument of the present invention. The measuring instrument is configured to measure the flow property with the capillary tube being tilted. It is possible to have a configuration conforming to the measuring instrument 101 in Figure 1 except that a test liquid is all housed in the capillary tube and the capillary tube is tilted. In this example, the capillary tube is disposed on a tilted stage, and a liquid column of the test liquid between the right end R on the second opening part side and the left end L on the first opening part side is included in the capillary tube. Sensors 1 to 3 that detect an end part of the liquid column are disposed along the capillary tube. The left end of the capillary tube is connected to the pressure adjustment unit, and the pressure control unit is controlled by the control unit. When the sensor 3 has detected an end part of the liquid column, flow of the test liquid in the capillary tube can be stopped by closing a space around a pipe through control of the adjustment valve or the like connected to the pressure adjustment unit.

The tilt of the capillary tube in Figure 12 can be adjusted as appropriate, but an angle θ between the horizontal direction and the axis of the capillary tube may be set to approximately 10° to 60°, 15° to 50°, or 20° to 40° from the perspectives of, for example, easily performing measurement including influence of the tilt and easily controlling flow of the test liquid in the capillary tube as appropriate by using the adjustment valve and the like.

In this example, the capillary tube is tilted relative to the horizontal direction. The angle of the capillary tube relative to the horizontal direction is represented by θ. Furthermore, the electromagnetic valve is connected to the lower end of the capillary tube and closed.

A specimen of the test liquid (for example, approximately 5 µL) is injected from the upper end of the capillary tube. The specimen all enters the capillary tube and comes to rest at a position in balance with the internal pressure.

Thereafter, when the electromagnetic valve is opened to open the inside of the capillary tube to the atmospheric pressure, the test liquid in a liquid column shape in the capillary tube falls inside the capillary tube in accordance with gravitational force. Flow speed v of the liquid column is expressed by Expression (4).
[Math. 4] $v = \frac{{D}^{2} ρg}{32 η} sin θ$ (4)

Symbols used in Expression (4) are as follows.
v: flow speed (mm/ms) of the liquid column
D: inner diameter (mm) of the capillary tube
ρ: density (kg/m³) of the liquid specimen
η: viscosity coefficient (mPa·s) of the liquid specimen
g: gravitational acceleration (m/s²)
θ: angle of the capillary tube relative to the horizontal direction

When the capillary tube inner diameter, the gravitational acceleration, and the tilt angle are constant, Expression (4) can be rewritten as Expression (5) below.
[Math. 5] $v = K \left(D, g, θ\right) {\left(\frac{η}{ρ}\right)}^{- 1}$ (5)

The coefficient K(D, g, θ) in Expression (5) is a constant determined by the capillary tube inner diameter, the gravitational acceleration, and the tilt angle of the capillary tube. Accordingly, the flow speed v of the liquid is inversely proportional to the kinetic viscosity η/ρ of the liquid specimen. Note that the flow speed v does not depend on the length (that is, injection amount) of the liquid column.

A time in which the liquid column proceeds a constant distance d when the capillary tube inner diameter, the gravitational acceleration, and the tilt angle are constant is represented by Δt. In this case, since passing time is inversely proportional to speed, Expression (5) can be rewritten as Expression (6) below.
[Math. 6] $Δ t = L \left(D, g, θ, d\right) \left(\frac{η}{ρ}\right)$ (6)

L(D, g, θ, d) is a constant determined by the capillary tube inner diameter, the gravitational acceleration, the tilt angle of the capillary tube, and the distance d. Accordingly, the flow time Δt of the liquid is proportional to the kinetic viscosity η/ρ of the liquid specimen. Note that, in this case as well, the flow time Δt does not depend on the length (that is, injection amount) of the liquid column.

In Expression (6), the coefficient L(D, g, θ, d) is a constant determined by the capillary tube inner diameter, the gravitational acceleration, the tilt angle of the capillary tube, and the distance d. Furthermore, the flow time does not depend on the injection amount (length of the liquid column) under a condition that the length of the liquid column is shorter than the length of the capillary tube. Accordingly, Expression (7) below holds even if the injection amount differs, where the flow time of a first specimen (1) is represented by Δt₁ and the flow time of a second specimen (2) is represented by Δt₂. Thus, the ratio of the flow time of the liquid specimen is equal to the ratio of the kinetic viscosity. Note that, in Expression (7), η_{1/}ρ₁ is the kinetic viscosity of the first specimen, and η_{2/}ρ₂ is the kinetic viscosity of the second specimen.
[Math. 7] $\frac{Δ {t}_{2}}{Δ {t}_{1}} = \frac{\left(\frac{{η}_{2}}{{ρ}_{2}}\right)}{\left(\frac{{η}_{1}}{{ρ}_{1}}\right)}$ (7)

### [Sixth Embodiment]

The present invention may have a sixth embodiment that the pressure adjustment unit further has a configuration capable of adjusting pressure in stages. Figure 13 is an overview diagram related to the pressure difference of a capillary tube according to the present invention and the flow property.

A case where the inside of the capillary tube is set to pressure different from the atmospheric pressure is assumed and this form will be described below. The atmospheric pressure is represented by P0, and the capillary tube internal pressure at a time point when the electromagnetic valve is opened is represented by P. The pressure difference is represented by ΔP = P - P0. Expression (8) holds when the other conditions are the same as in the fifth embodiment.
[Math. 8] $v = - \frac{{D}^{2}}{32 ηl} Δ P + \frac{{D}^{2} ρg}{32 η} sin θ$ (8)

Symbols used in Expression (8) are as follows.
v: flow speed (mm/ms) of the liquid column
D: inner diameter (mm) of the capillary tube
ΔP: pressure difference (Pa) between the ends of the liquid column
ρ: density (kg/m³) of the liquid specimen
η: viscosity coefficient (mPa·s) of the liquid specimen
l: length (mm) of the examination liquid housed in the tube
g: gravitational acceleration (m/s²)
θ: angle of the capillary tube relative to the horizontal direction

Figure 13 is plotted with the pressure difference ΔP on the horizontal axis and the speed v on the vertical axis. With ΔP = 0, Expression (8) reduces to Expression (4). In other words, the value of the y-intercept of the plot is equal to the speed value in Expression (4).

The capillary tube is tilted to cause the liquid specimen to fall with the pressure difference ΔP created between the inside and outside of the capillary tube. A right downward straight line as in Figure 13 is obtained by plotting with the fall speed v of the liquid column on the vertical axis and the pressure difference ΔP on the horizontal axis. The kinetic viscosity η/ρ of the specimen can be determined from a point (y-intercept) where the straight line intersects the vertical axis. The density ρ of the specimen can be determined from a point (x-intercept) where the straight line intersects the horizontal axis. The value ΔP_{c2} = lρgsinθ of the x-intercept of the plot is named as the critical pressure in the sixth embodiment of the present invention. The test liquid in the tilted capillary tube is at rest under the critical pressure. The test liquid in the capillary tube rises against the tilt when the positive pressure larger than the critical pressure is applied in the capillary tube.

The specimen having fallen to and stopped at the lowermost end of the capillary tube can be pushed back to the upper end side (fall start position) by applying a pressure difference larger than the critical pressure ΔP_{c2}. As an example, the value of ΔP_{c2} is approximately 50 Pa in a case where the test liquid is pure water of 5 µL and the tilt angle is 30°. Thus, to complete the plot of Figure 13 by repeating fall measurement, the pressure difference of 100 Pa or less is produced with high reproducibility and performed.

### [Seventh embodiment]

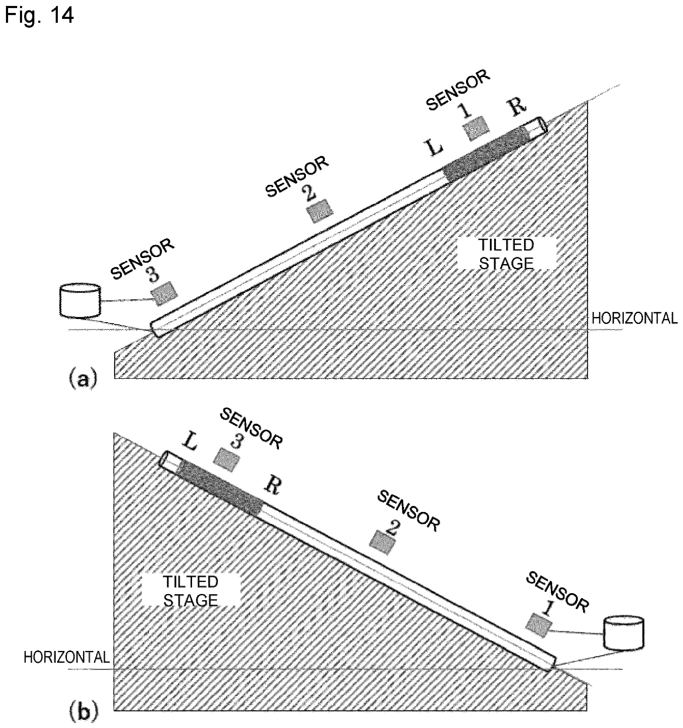

Figure 14 is an overview diagram related to disposition and the like of a capillary tube according to a seventh embodiment of the measuring instrument of the present invention. The push-back means may include a tilted stage for disposing the capillary tube and may be installed in a configuration where the first opening part side is positioned lower than the second opening part for flowing and in a configuration where the second opening part side is positioned lower than the first opening part for pushing back a test liquid in the capillary tube.

When the tilt angle of the capillary tube after flow measurement end is inverted as in Figure 14, the specimen in the capillary tube can be caused to flow back by the effect of gravitational force without pressure application and can be pushed back to start position. This method of inverting the tilt angle for returning to the initial state further adds tilt measurement because motion at the inversion is equivalent to one with the inverted signs of v and θ in Expression (4). This configuration does not necessarily need pressure adjustment of the positive pressure for pushing back. Note that an adjustment valve for adjusting pressure is preferably attached to the first opening part and/or the second opening part so that the liquid column of the test liquid can be stopped in the capillary tube.

### Example

The present invention will be described further in detail below with an example.

### [Measuring device]

- Capillary tube: a capillary tube with a radius r of 0.34 mm (nominal value) was used. The length thereof is 100 mm.
- The negative pressure and the positive pressure were adjusted by using a container (specimen bottle of 1000 mL) and a variable-capacity container (digital pipette), which correspond to the pressure adjustment unit 3.
- Pressure was measured by a pressure meter and calibrated.
- An electromagnetic valve was used as the switching valve.

- Sensors: a plurality of optical sensors for detecting the liquid level position were disposed along the capillary tube.
- Liquid reservoir part: a placement table provided with a concave part so that the leading end of the capillary tube contacts was used.
- Standard solution: pure water was used.
- Sucrose solution: a mixture of pure water with a sucrose concentration of 30 mass% (sucrose water solution of 30 mass%) was used.
- Room temperature is 21°C ± 0.5°C.

### [Test example 1]

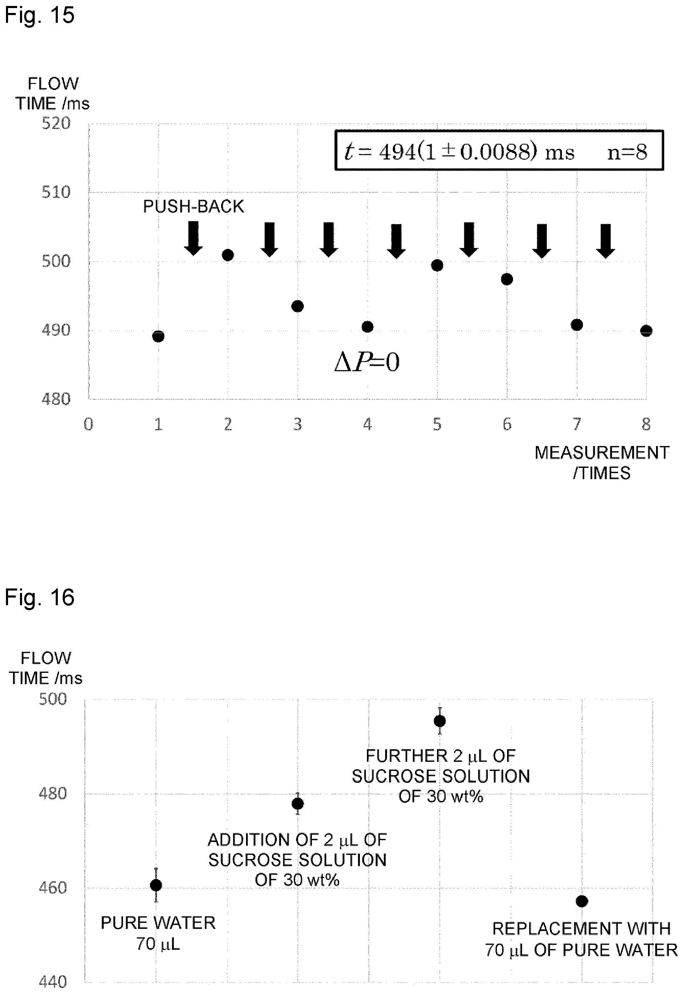

A test example 1 to a test example 4 are each a test example in the first embodiment. Time of passing through a flow path of 60 mm at the atmospheric pressure was measured by using pure water as a test liquid. When the liquid level passed by measurement distance and moved to a position close to the first opening part, the test liquid was stopped, the pressure adjustment unit was switched to the push-back pressure, and the switching valve was switched to push back the test liquid to a stop position in the vicinity of the second opening part. Thereafter, the first opening part side was switched to the inflow direction again to perform measurement. Measured results of variance when the test was performed eight times are illustrated in Figure 15. Through the eight times of the test, it was checked that stable results were obtained.

### [Test example 2]

The test was performed according to the test example 1 while the state of the test liquid in the liquid reservoir part was changed at flow reciprocation. First, at the first inflow, a test of the flow time was performed with pure water eight times. Subsequently, the liquid was pushed back without bubbling at a position where only a small amount of the liquid remains in the capillary tube, and before the second inflow, 2 µL of the sucrose solution of 30 mass% was added to the liquid reservoir part to change the property of the liquid, and then a test of the flow time was performed eight times. Subsequently, in the third test according to the second test, further 2 µL of the sucrose solution of 30 mass% was added to the liquid reservoir part to change the property of the liquid, and then a test of the flow time was performed nine times. Thereafter, before the fourth inflow, the liquid in the liquid reservoir was replaced with pure water, and a test of the flow time was performed nine times. Measurement results of the flow time in each test are illustrated as an average value and a standard deviation (length of an error bar) in Figure 16. Change of the flow time along with slight change of the liquid in the liquid reservoir was measured and it was checked that measurement with high reproducibility of the first result for pure water can be performed through replacement with pure water.

### [Test example 3]

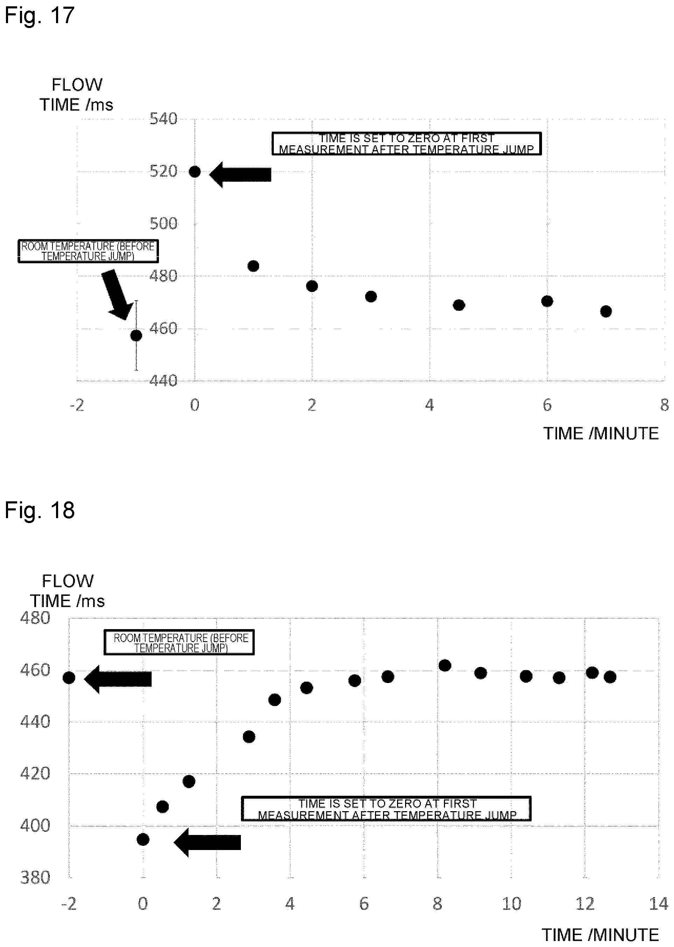

Measurement of the flow property along with liquid temperature change was performed. First, at room temperature, 70 µL of pure water was disposed in the liquid reservoir part and the flow property was measured. After a test, a liquid in the capillary tube was pushed back. Subsequently, 50 µL of ice-cooled pure water was mixed to the liquid reservoir part and the same amount of pure water, 50 µL, was taken out. Furthermore, in repetition, 50 µL of ice-cooled pure water was mixed to the liquid reservoir part and the same amount of pure water, 50 µL, was taken out. Thereafter, the flow property at inflow to the capillary tube was measured immediately. Push-back was performed as soon as the measurement at inflow ended. The flow time of inflow was measured approximately every one minute. Results of the measurement are illustrated in Figure 17. It was checked that difference in the flow time along with temperature change of the test liquid can be measured. Furthermore, in this measurement, it is thought that liquid temperature immediately returns to room temperature, but it was checked that it was possible to trace and measure flow property displacement that occurs in such a short time of several minutes.

### [Test example 4]

Measurement of the flow property along with liquid temperature change was performed. First, at room temperature, 70 µL of pure water was disposed in the liquid reservoir part and the flow property was measured. After a test, a liquid in the capillary tube was pushed back. Subsequently, 50 µL of pure water at 50°C was mixed to the liquid reservoir part and the same amount of pure water, 50 µL, was taken out. Furthermore, in repetition, 50 µL of pure water at 50°C was mixed to the liquid reservoir part and the same amount of pure water, 50 µL, was taken out. Thereafter, the flow property at inflow to the capillary tube was measured immediately. Push-back was performed as soon as the measurement at inflow ended. The flow time of inflow was measured approximately every one minute. Results of the measurement are illustrated in Figure 18. It was checked that difference in the flow time along with temperature change of the test liquid can be measured. Furthermore, in this measurement, it is thought that liquid temperature relatively gradually returns to room temperature as compared to the test example 3 at low temperature, but it was checked that it was possible to trace and measure flow property displacement that occurs in such a short time of several minutes.

### [Test example 5]

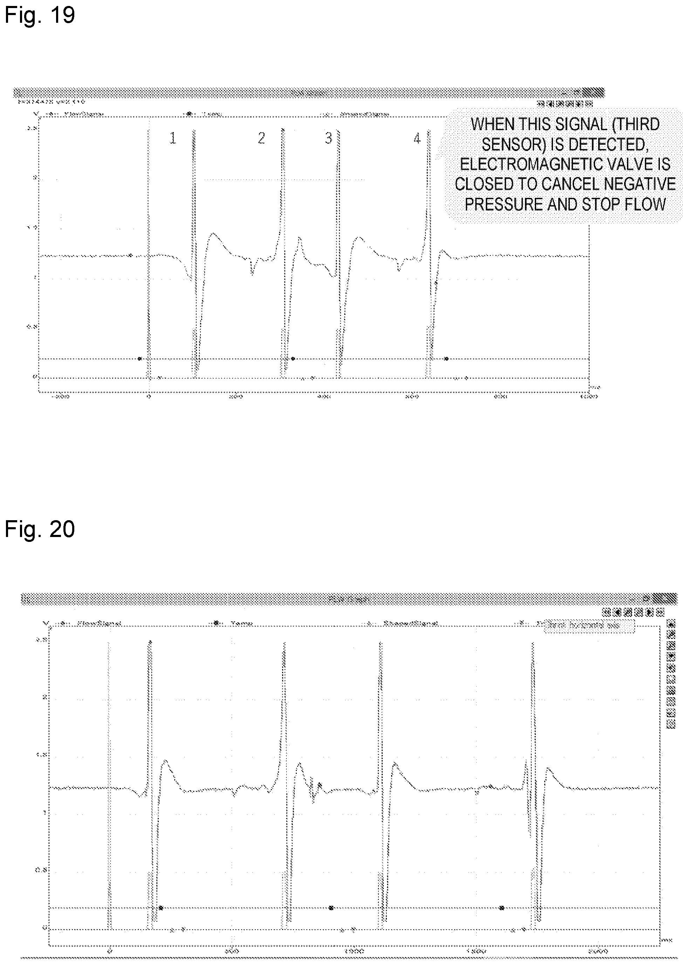

Measurement was performed to check that the flow time is proportional to the viscosity in a case where the test liquid is all housed in the capillary tube (corresponding to Figure 8). Five µL of pure water was administered to the capillary tube horizontally placed and was caused to flow under application of the negative pressure (97 Pa) (Figure 19).

A signal diagram of Figure 19 has the following features.
- Signal 1 is output when the R end of an examination liquid passes through a first sensor; time point is t1.
- Signal 2 is output when the L end of the examination liquid passes through a second sensor; time point is t2.
- Signal 3 is output when the R end of the examination liquid passes through the second sensor; time point is t3.
- Signal 4 is output when the L end of the examination liquid passes through a third sensor; time point is t4.

In this measurement, the inter-sensor distance was fixed to 30 mm. Thus, speed at which the liquid column moves in the capillary tube can be calculated from (t3 - t1) or (t4 - t2).

After the measurement with pure water, a specimen as the examination liquid in the capillary tube was pushed out, 5 µL of the sucrose solution of 30 mass% was newly administered and reciprocated in the capillary tube for rinsing. After the rinsing, the sucrose solution was pushed out and 5 µL of the sucrose solution of 30 mass% was administered again. Figure 20 illustrates a signal obtained by flow recording with the negative pressure of 97 Pa applied to this specimen. The flow time of the sucrose solution of 30 mass% was 3.0 times longer than that of pure water. This value is substantially equal to a value expected from the viscosity ratio 3.17 of the sucrose solution and pure water.

### [Test example 6]

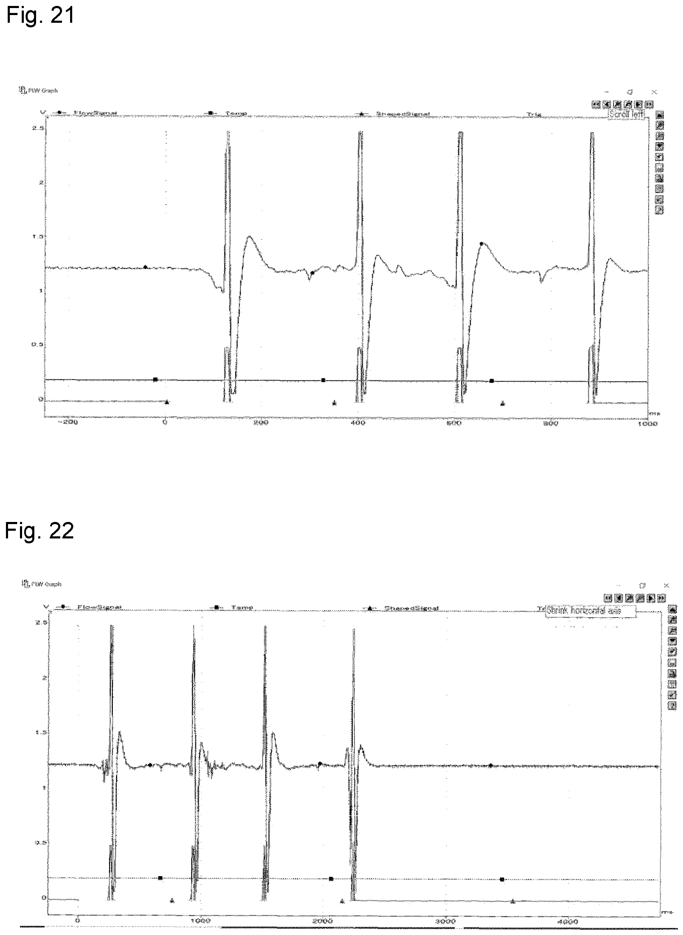

Figure 21 is a graph of measurement results for pure water in the example of the present invention. Figure 22 is a graph of measurement results for the sucrose solution of 30 mass% in the example of the present invention. These measurements are examples in which the flow property of the test liquid was evaluated according to the fifth embodiment with the configuration in Figure 12. The measurements are suitable for, for example, measurement of kinetic viscosity, in particular.

The capillary tube was tilted as in Figure 12 with sinθ = 0.57. Five µL of pure water was injected into the capillary tube, the electromagnetic valve was opened, and the flow time of the liquid column was recorded (Figure 21).

After signal recording, the positive pressure was applied to push the pure water in the capillary tube out of the tube, 5 µL of the sucrose solution of 30 mass% was injected and then discharged after rinsing. Five µL of the sucrose solution of 30 mass% was newly injected and the flow time was recorded (Figure 22).

In Figures 21 and 22 of signal recording, the first signal indicates a time point when the R end of the liquid column passes through the first sensor, the second signal indicates a time point when the L end of the liquid column passes through the second sensor, the third signal indicates a time point when the R end of the liquid column passes through the second sensor, and the fourth signal indicates a time point when the L end of the liquid column passes through the third sensor.

A specimen is injected and preparation is performed for measurement. Sensors are the sensors 1, 2, and 3 in order from the right side (closer to the second opening part as an entrance). Five µL of the specimen is injected. The specimen is all sucked by surface tension. Then, the specimen is caused to fall. When passing of the test liquid is sensed by the sensor 3, the electromagnetic valve is closed to stop the specimen liquid.

Thereafter, the positive pressure is applied for pushing back. Upon sensing by the sensor 1, the positive pressure is canceled to stop the specimen liquid. The specimen stops immediately after passing through the sensor 1.

When the flow property is to be evaluated, the test liquid is caused to fall from a state in which the liquid column of the test liquid is located on the upper end side as in Figure 12. Signal 1 is output when the R end of the specimen passes through the first sensor; time point is t1. Signal 2 is output when the L end of the specimen passes through the second sensor; time point is t2. Signal 3 is output when the R end of the specimen passes through the second sensor; time point is t3. Signal 4 is output when the L end of the specimen passes through the third sensor; time point is t4. The fall time (t3 - t1) or (t4 - t2) is proportional to the kinetic viscosity. Note that this measurement does not depend on the injection amount in the capillary tube.

Note that, according to literature values, the density of the sucrose solution of 30 mass% is 1.127 times higher than that of pure water (20°C). Furthermore, the viscosity of the sucrose solution of 30 wt% is 3.17 times larger than that of pure water. Accordingly, the kinetic viscosity of the sucrose solution of 30 mass% is 2.81 times larger than that of pure water.

Results based on actual values of a test with this configuration indicated that the flow time of the sucrose solution of 30 mass% was 2.7 times longer than that of pure water. Thus, it was checked that the kinetic viscosities of two kinds of liquid specimens in an extremely small amount (5 µL) can be determined by comparison at the accuracy of 5% or lower.

### Industrial Applicability

The present invention can be used for measurement of a flow property such as the viscosity of liquid and is useful in industry.

### Reference Signs List

101 measuring instrument
11 capillary tube
111 first opening part
112 second opening part
2 switching valve
21 to 23 flow path
3 pressure adjustment unit
30 container
31 pressure meter
32 adjustment piston
4 liquid reservoir part
5 test liquid
6 measurement unit
60 timing unit
61 to 66 sensor
69 push-back detection unit
7 placement table
8 control unit
91 display unit

## Claims

1. A method for measuring a flow property of a test liquid (5) by using a measuring instrument (101) comprising:
a push-back means configured to push back the test liquid (5), the test liquid (5) having flowed into a capillary tube (11) including a first opening part (111) and a second opening part (112) from the second opening part (112) side toward the first opening part (111) side, to the second opening part (112) side;
an adjustment valve for adjusting liquid flow in the capillary tube (11); and
a measurement unit (6) configured to measure an arrival time tn at a liquid level position Ln in the capillary tube (11), and **(A)** or **(B):**
**(A)** the push-back means being a pressure adjustment unit (3) that is connected to the first opening part (111) side and can be set to pressure with which the test liquid (5) having flowed into the capillary tube (11) is pushed back to the second opening part (112) side,
the adjustment valve including a switching valve (2) for switching connection between the pressure adjustment unit (3) and the capillary tube (11),
the measuring instrument (101) including a detection unit configured to measure a flow property of a test liquid (5) by using a measuring instrument (101) including a push-back detection unit (69) configured to detect that the test liquid (5) in the capillary tube (11) has been pushed back to the second opening part (112) side and passed through, or
**(B)** the push-back means including a tilted stage for disposing the capillary tube (11) and being able to be installed in a configuration where the first opening part (111) side is positioned lower than the second opening part (112) for flowing and in a configuration where the second opening part (112) side is positioned lower than the first opening part (111) for pushing back the test liquid (5) in the capillary tube (11).

2. The method according to claim 1, comprising a flow property of the test liquid (5) of **(A)** housed inside the capillary tube (11) in an amount less than volume capacity of the capillary tube (11) being measured, and
the method including a second push-back process that the pressure adjustment unit (3) is connected to the first opening part (111) with pressure for push-back to the second opening part (112) side, the test liquid (5) housed in the capillary tube (11) is pushed back to the second opening part (112) side, and push-back is stopped when the test liquid (5) is pushed back to the push-back detection unit (69).

3. The method according to claim 1 or 2, wherein the pressure adjustment unit (3) of **(A)** includes a container (30), an adjustment piston (32) connected to the container (30), and a mechanism configured to open the container (30) to atmospheric pressure and hold inside of the container (30) at atmospheric pressure until right before isothermal compression and/or isothermal expansion in coordination with the switching valve (2), and
when pressure inside the container (30) is to be adjusted, a predetermined pressure inside the container (30) is adjusted through isothermal compression and/or isothermal expansion by changing volumes of the container (30) and the adjustment piston (32) by using the adjustment piston (32) after isolation of the container (30) from atmosphere.

4. The method according to claim 1, wherein the measuring instrument (101) of **(A)**
includes a liquid reservoir part (4) that contacts the second opening part (112) side, and
measures a flow property of a test liquid (5) in a capillary tube (11), the test liquid (5) being housed in the liquid reservoir part (4) having flowed in from the second opening part (112),
the method including a first push-back process that the pressure adjustment unit (3) is connected to the first opening part (111) with a positive pressure relative to a critical pressure, the test liquid (5) having flowed into the capillary tube (11) being sent from the second opening part (112) to the liquid reservoir part (4), and push-back being stopped when the test liquid (5) is pushed back to the push-back detection unit (69), and
the method including, after the first push-back process, a process of measuring a flow property when the test liquid (5) in the liquid reservoir part (4) has been mixed with a test target component and caused to flow into the capillary tube (11).

5. The method according to claim 3, wherein the measuring instrument (101) of **(A)**
includes a liquid reservoir part (4) that contacts the second opening part (112) side, and
measures a flow property of a test liquid (5) in a capillary tube (11), the test liquid (5) being housed in the liquid reservoir part (4) having flowed in from the second opening part (112), and
the method includes a first push-back process that the pressure adjustment unit (3) is connected to the first opening part (111) with a positive pressure relative to a critical pressure, the test liquid (5) having flowed into the capillary tube (11) is sent from the second opening part (112) to the liquid reservoir part (4), and push-back is stopped when the test liquid (5) is pushed back to the push-back detection unit (69).

6. The method according to claim 5, comprising, after the first push-back process, a process of measuring a flow property when the test liquid (5) in the liquid reservoir part (4) has been mixed with a test target component and caused to flow into the capillary tube (11).

7. The method according to any one of claims 2, 3, 5 or 6, wherein the capillary tube (11) is tilted.

8. The method according to claim 7, wherein the pressure adjustment unit (3) can further adjust pressure in stages.

9. A system for measuring a flow property of a test liquid (5), the system includes a measuring instrument (101) comprising:
a push-back means configured to push back the test liquid (5), the test liquid (5) having flowed into a capillary tube (11) including a first opening part (111) and a second opening part (112) from the second opening part (112) side toward the first opening part (111) side, to the second opening part (112) side;
an adjustment valve for adjusting liquid flow in the capillary tube (11); and
a measurement unit (6) configured to measure an arrival time tn at a liquid level position Ln in the capillary tube (11), and **(A)** or **(B):**
**(A)** the push-back means being a pressure adjustment unit (3) that is connected to the first opening part (111) side and can be set to pressure with which the test liquid (5) having flowed into the capillary tube (11) is pushed back to the second opening part (112) side,
the adjustment valve including a switching valve (2) for switching connection between the pressure adjustment unit (3) and the capillary tube (11),
the measuring instrument (101) including a detection unit configured to measure a flow property of a test liquid (5) by using a measuring instrument (101) including a push-back detection unit (69) configured to detect that the test liquid (5) in the capillary tube (11) has been pushed back to the second opening part (112) side and passed through, or
**(B)** the push-back means including a tilted stage for disposing the capillary tube (11) and being able to be installed in a configuration where the first opening part (111) side is positioned lower than the second opening part (112) for flowing and in a configuration where the second opening part (112) side is positioned lower than the first opening part (111) for pushing back the test liquid (5) in the capillary tube (11).

10. The system according to claim 9, wherein the measuring instrument (101) of **(A)**
includes a liquid reservoir part (4) that contacts the second opening part (112) side, and
measures a flow property of a test liquid (5) in a capillary tube (11), the test liquid (5) being housed in the liquid reservoir part (4) having flowed in from the second opening part (112),
the measuring instrument (101) including a first push-back process means that the pressure adjustment unit (3) is connected to the first opening part (111) with a positive pressure relative to a critical pressure, the test liquid (5) having flowed into the capillary tube (11) being sent from the second opening part (112) to the liquid reservoir part (4), and push-back being stopped when the test liquid (5) is pushed back to the push-back detection unit (69), and
the measuring instrument (101) including, after the first push-back process, a means for measuring a flow property when the test liquid (5) in the liquid reservoir part (4) has been mixed with a test target component and caused to flow into the capillary tube (11).

## Patentansprüche

1. Verfahren zum Messen einer Fließeigenschaft einer Testflüssigkeit (5) unter Verwendung eines Messgeräts (101) umfassend:
eine Rückdruckvorrichtung, die so ausgelegt ist, dass sie die Testflüssigkeit (5), die in ein Kapillarrohr (11) mit einem ersten Öffnungsabschnitt (111) und einem zweiten Öffnungsabschnitt (112) von der Seite des zweiten Öffnungsabschnitts (112) in Richtung der Seite des ersten Öffnungsabschnitts (111) geflossen ist, zur Seite des zweiten Öffnungsabschnitts (112) zurückdrückt;
ein Einstellventil zum Einstellen von Flüssigkeitsstrom in dem Kapillarrohr (11); und
eine Messeinheit (6), die so ausgelegt ist, dass sie eine Ankunftszeit tn an einer Flüssigkeitsstandposition Ln in dem Kapillarrohr (11) misst, und **(A)** oder **(B):**
**(A)** wobei die Rückdruckvorrichtung eine Druckeinstellungseinheit (3) ist, die mit der Seite des ersten Öffnungsabschnitts (111) verbunden ist und auf einen Druck eingestellt werden kann, mit dem die in das Kapillarrohr (11) geflossene Testflüssigkeit (5) zur Seite des zweiten Öffnungsabschnitts (112) zurückgedrückt ist,
wobei das Einstellventil ein Umschaltventil (2) zum Umschalten der Verbindung zwischen der Druckeinstellungseinheit (3) und dem Kapillarrohr (11) umfasst,
wobei das Messgerät (101) eine Erfassungseinheit umfasst, die so ausgelegt ist, dass sie eine Fließeigenschaft einer Testflüssigkeit (5) misst, indem sie ein Messgerät (101) umfassend eine Rückdruckerfassungseinheit (69) verwendet, die so ausgelegt ist, dass sie erfasst, dass die Testflüssigkeit (5) im Kapillarrohr (11) zur Seite des zweiten Öffnungsabschnitts (112) zurückgedrückt wurde und durchgelaufen ist, oder
**(B)** wobei die Rückdruckvorrichtung einen geneigten Aufbau zum Anordnen des Kapillarrohrs (11) umfasst und so angelegt werden kann, dass die Seite des ersten Öffnungsabschnitts (111) tiefer liegt als die Seite des zweiten Öffnungsabschnitts (112) für den Durchfluss, sowie so angelegt werden kann, indem die Seite des zweiten Öffnungsabschnitts (112) tiefer liegt als der erste Öffnungsabschnitt (111), um die Testflüssigkeit (5) im Kapillarrohr (11) zurückzudrängen.

2. Verfahren gemäß Anspruch 1, bei dem eine Fließeigenschaft der Testflüssigkeit (5) gemessen wird, die in einer Menge von **(A)** innerhalb des Kapillarrohrs (11) vorhanden ist, die geringer ist als das Fassungsvermögen des Kapillarrohrs (11), und
das Verfahren einen zweiten Rückdruckvorgang umfasst, bei dem die Druckeinstellungseinheit (3) mit dem ersten Öffnungsabschnitt (111) mit Druck zum Rückdruck zur Seite des zweiten Öffnungsabschnitts (112) verbunden ist, die im Kapillarrohr (11) befindliche Testflüssigkeit (5) zur Seite des zweiten Öffnungsabschnitts (112) zurückgedrückt ist und das Zurückdrücken gestoppt ist, wenn die Testflüssigkeit (5) bis zur Rückdruckerfassungseinheit (69) zurückgedrückt ist.

3. Verfahren gemäß Anspruch 1 oder 2, wobei die Druckeinstellungseinheit (3) von **(A)** einen Behälter (30), einen mit dem Behälter (30) verbundenen Einstellkolben (32), sowie einen Mechanismus, der so ausgelegt ist, dass er den Behälter (30) auf Atmosphärendruck öffnet und innerhalb des Behälters (30) bis unmittelbar vor der isothermen Kompression und/oder isothermen Expansion in Abstimmung mit dem Umschaltventil (2) auf Atmosphärendruck hält, und
wenn Druck innerhalb des Behälters (30) eingestellt werden soll, ein vorbestimmter Druck innerhalb des Behälters (30) durch isotherme Kompression und/oder isotherme Expansion eingestellt ist, indem Volumen des Behälters (30) und des Einstellkolbens (32) unter Verwendung des Einstellkolbens (32) nach Isolierung des Behälters (30) von der Atmosphäre verändert werden.

4. Verfahren gemäß Anspruch 1, wobei das Messgerät (101) von **(A)**
einen Flüssigkeitsreservoirabschnitt (4) umfasst, der an der Seite des zweiten Öffnungsabschnitts (112) anliegt, und
eine Fließeigenschaft einer Testflüssigkeit (5) in einem Kapillarrohr (11) misst, wobei die Testflüssigkeit (5), die in dem Flüssigkeitsreservoirabschnitt (4) aufgenommen ist, aus dem zweiten Öffnungsabschnitt (112) eingeströmt ist,
wobei das Verfahren einen ersten Rückdruckvorgang umfasst, bei dem die Druckeinstellungseinheit (3) mit dem ersten Öffnungsabschnitt (111) unter einem Überdruck relativ zu einem kritischen Druck verbunden ist, wobei die in das Kapillarrohr (11) eingeströmte Testflüssigkeit (5) vom zweiten Öffnungsabschnitt (112) zum Flüssigkeitsreservoirabschnitt (4) geleitet ist, und der Rückdruck gestoppt wird, wenn die Testflüssigkeit (5) bis zur Rückdruckerfassungseinheit (69) zurückgedrückt ist, und
das Verfahren nach dem ersten Rückdruckvorgang einen Vorgang zum Messen einer Fließeigenschaft umfasst, wenn die Testflüssigkeit (5) in dem Flüssigkeitsreservoirabschnitt (4) mit einer zu untersuchenden Zielkomponente vermischt und dazu gebracht worden ist, in das Kapillarrohr (11) zu fließen.

5. Verfahren gemäß Anspruch 3, wobei das Messgerät (101) von **(A)**
einen Flüssigkeitsreservoirabschnitt (4) umfasst, der an der Seite des zweiten Öffnungsabschnitts (112) anliegt, und
eine Fließeigenschaft einer Testflüssigkeit (5) in einem Kapillarrohr (11) misst, wobei die im Flüssigkeitsreservoirabschnitt (4) befindliche Testflüssigkeit (5) aus dem zweiten Öffnungsabschnitt (112) eingeströmt ist, und
das Verfahren einen ersten Rückdruckvorgang umfasst, bei dem die Druckeinstellungseinheit (3) mit dem ersten Öffnungsabschnitt (111) unter einem Überdruck relativ zu einem kritischen Druck verbunden ist, wobei die in das Kapillarrohr (11) geflossene Testflüssigkeit (5) vom zweiten Öffnungsabschnitt (112) zum Flüssigkeitsreservoirabschnitt (4) geleitet ist, und Rückdruck gestoppt ist, wenn die Testflüssigkeit (5) bis zur Rückdruckerfassungseinheit (69) zurückgedrückt ist.

6. Verfahren gemäß Anspruch 5, das nach dem ersten Rückdruckvorgang einen Vorgang zum Messen einer Fließeigenschaft umfasst, wenn die Testflüssigkeit (5) im Flüssigkeitsreservoirabschnitt (4) mit einer zu untersuchenden Zielkomponente vermischt und dazu gebracht worden ist, in das Kapillarrohr (11) zu fließen.

7. Verfahren gemäß einem der Ansprüche 2, 3, 5 oder 6, wobei das Kapillarrohr (11) geneigt ist.

8. Verfahren gemäß Anspruch 7, wobei die Druckeinstellungseinheit (3) den Druck ferner stufenweise einstellen kann.

9. System zur Messung einer Fließeigenschaft einer Testflüssigkeit (5), wobei das System ein Messgerät (101) umfasst, das Folgendes umfasst:
eine Rückdruckvorrichtung, die so ausgelegt ist, dass sie die Testflüssigkeit (5), die in ein Kapillarrohr (11) mit einem ersten Öffnungsabschnitt (111) und einem zweiten Öffnungsabschnitt (112) von der Seite des zweiten Öffnungsabschnitts (112) in Richtung der Seite des ersten Öffnungsabschnitts (111) geflossen ist, in Richtung der Seite des zweiten Öffnungsabschnitts (112) zurückdrückt;
ein Einstellventil zum Einstellen des Flüssigkeitsstroms im Kapillarrohr (11); und
eine Messeinheit (6), die so ausgelegt ist, dass sie eine Ankunftszeit tn an einer Flüssigkeitsstandposition Ln im Kapillarrohr (11) misst, und **(A)** oder **(B):**
**(A)** wobei die Rückdruckvorrichtung eine Druckeinstellungseinheit (3) ist, die mit der Seite des ersten Öffnungsabschnitts (111) verbunden ist und auf einen Druck eingestellt werden kann, mit dem die in das Kapillarrohr (11) geflossene Testflüssigkeit (5) zur Seite des zweiten Öffnungsabschnitts (112) zurückgedrückt ist,
wobei das Einstellventil ein Umschaltventil (2) zum Umschalten der Verbindung zwischen der Druckeinstellungseinheit (3) und dem Kapillarrohr (11) umfasst,
wobei das Messgerät (101) eine Erfassungseinheit umfasst, die so ausgelegt ist, dass sie eine Fließeigenschaft einer Testflüssigkeit (5) durch Verwenden eines Messgeräts (101) umfassend eine Rückdruckerfassungseinheit (69) misst, die so ausgelegt ist, dass sie erfasst, dass die Testflüssigkeit (5) im Kapillarrohr (11) zur Seite des zweiten Öffnungsabschnitts (112) zurückgedrückt wurde und durchgelaufen ist, oder
**(B)** die Rückdruckvorrichtung umfasst einen geneigten Aufsatz zum Anordnen des Kapillarrohrs (11) und kann so ausgelegt werden, indem die Seite des ersten Öffnungsabschnitts (111) tiefer liegt als die des zweiten Öffnungsabschnitts (112) für den Durchfluss, und so ausgelegt werden kann, indem die Seite des zweiten Öffnungsabschnitts (112) tiefer liegt als der erste Öffnungsabschnitt (111), um die Testflüssigkeit (5) im Kapillarrohr (11) zurückzudrücken.

10. System gemäß Anspruch 9, wobei das Messgerät (101) von **(A)**
einen Flüssigkeitsreservoirabschnitt (4) umfasst, der an der Seite des zweiten Öffnungsabschnitts (112) anliegt, und
eine Fließeigenschaft einer Testflüssigkeit (5) in einem Kapillarrohr (11) misst, wobei die im Flüssigkeitsvorratsabschnitt (4) befindliche Testflüssigkeit (5) vom zweiten Öffnungsabschnitt (112) her eingeströmt ist,
wobei das Messgerät (101) einen erste Rückdruckvorgang umfasst, bedeutend, dass die Druckeinstellungseinheit (3) mit dem ersten Öffnungsabschnitt (111) unter einem Überdruck relativ zu einem kritischen Druck verbunden ist, wobei die in das Kapillarrohr (11) eingeströmte Testflüssigkeit (5) vom zweiten Öffnungsabschnitt (112) zum Flüssigkeitsreservoirabschnitt (4) geleitet ist, und Rückdruck gestoppt ist, wenn die Testflüssigkeit (5) bis zur Rückdruckerfassungseinheit (69) zurückgedrückt ist, und
das Messgerät (101) nach dem ersten Rückdruckvorgang eine Vorrichtung zum Messen einer Fließeigenschaft umfasst, wenn die Testflüssigkeit (5) im Flüssigkeitsreservoirabschnitt (4) mit einer zu untersuchenden Zielkomponente vermischt und dazu gebracht worden ist, in das Kapillarrohr (11) zu fließen.

## Revendications

1. Procédé de mesure d'une propriété d'écoulement d'un liquide d'essai (5) en utilisant un instrument de mesure (101) comprenant :
un moyen de refoulement configuré pour refouler le liquide d'essai (5), le liquide d'essai (5) ayant coulé dans un tube capillaire (11) incluant une première partie d'ouverture (111) et une seconde partie d'ouverture (112) depuis le côté seconde partie d'ouverture (112) vers le côté première partie d'ouverture (111), jusqu'au côté seconde partie d'ouverture (112) ;
une vanne de réglage pour régler le débit de liquide dans le tube capillaire (11) ; et
une unité de mesure (6) configurée pour mesurer une heure d'arrivée tn à une position de niveau de liquide Ln dans le tube capillaire (11), et **(A)** ou **(B):**
**(A)** le moyen de refoulement étant une unité de réglage de pression (3) qui est connectée au côté première partie d'ouverture (111) et peut être fixée à une pression avec laquelle le liquide d'essai (5) ayant coulé dans le tube capillaire (11) est refoulé vers le côté seconde partie d'ouverture (112),
la vanne de réglage incluant une vanne de commutation (2) pour commuter une connexion entre l'unité de réglage de pression (3) et le tube capillaire (11),
l'instrument de mesure (101) incluant une unité de détection configurée pour mesurer une propriété d'écoulement d'un liquide d'essai (5) en utilisant un instrument de mesure (101) incluant une unité de détection de refoulement (69) configurée pour détecter que le liquide d'essai (5) dans le tube capillaire (11) a été refoulé vers le côté seconde partie d'ouverture (112) et l'a traversé, ou
**(B)** le moyen de refoulement incluant un étage incliné pour disposer le tube capillaire (11) et pouvant être installé dans une configuration dans laquelle le côté première partie d'ouverture (111) est positionné plus bas que la seconde partie d'ouverture (112) pour l'écoulement et dans une configuration dans laquelle le côté seconde partie d'ouverture (112) est positionné plus bas que la première partie d'ouverture (111) pour refouler le liquide d'essai (5) dans le tube capillaire (11).

2. Procédé selon la revendication 1, comprenant une propriété d'écoulement du liquide d'essai (5) de **(A)** contenu à l'intérieur du tube capillaire (11) en une quantité inférieure à la capacité volumique du tube capillaire (11) qui doit être mesurée, et
le procédé incluant un second processus de refoulement dans lequel l'unité de réglage de pression (3) est connectée à la première partie d'ouverture (111) avec une pression pour refouler, vers le côté seconde partie d'ouverture (112), le liquide d'essai (5) logé dans le tube capillaire (11) est refoulé vers le côté seconde partie d'ouverture (112), et le refoulement est arrêté lorsque le liquide d'essai (5) est refoulé vers l'unité de détection de refoulement (69).

3. Procédé selon la revendication 1 ou 2, dans lequel l'unité de réglage de pression (3) de **(A)** inclut un récipient (30), un piston de réglage (32) relié au récipient (30) et un mécanisme configuré pour ouvrir le récipient (30) à la pression atmosphérique et maintenir l'intérieur du récipient (30) à pression atmosphérique jusqu'à juste avant une compression isotherme et/ou une détente isotherme en coordination avec la vanne de commutation (2), et
lorsque la pression à l'intérieur du récipient (30) doit être réglée, une pression prédéterminée à l'intérieur du récipient (30) est réglée par compression isotherme et/ou détente isotherme en modifiant les volumes du récipient (30) et du piston de réglage (32) en utilisant le piston de réglage (32) après isolation du récipient (30) de l'atmosphère.

4. Procédé selon la revendication 1, dans lequel l'instrument de mesure (101) de **(A)**
inclut une partie réservoir de liquide (4) qui vient en contact avec le côté seconde partie d'ouverture (112), et
mesure une propriété d'écoulement d'un liquide d'essai (5) dans un tube capillaire (11), le liquide d'essai (5) étant logé dans la partie réservoir de liquide (4) ayant coulé depuis la seconde partie d'ouverture (112),
le procédé incluant un premier processus de refoulement dans lequel l'unité de réglage de pression (3) est reliée à la première partie d'ouverture (111) avec une pression positive par rapport à une pression critique, le liquide d'essai (5) ayant coulé dans le tube capillaire (11) étant envoyé de la seconde partie d'ouverture (112) à la partie réservoir de liquide (4), et le refoulement étant arrêté lorsque le liquide d'essai (5) est refoulé vers l'unité de détection de refoulement (69), et
le procédé incluant, après le premier processus de refoulement, un processus de mesure d'une propriété d'écoulement lorsque le liquide d'essai (5) dans la partie réservoir de liquide (4) a été mélangé avec un composant cible d'essai et amené à s'écouler dans le tube capillaire (11).

5. Procédé selon la revendication 3, dans lequel l'instrument de mesure (101) de **(A)**
inclut une partie réservoir de liquide (4) qui vient en contact avec le côté seconde partie d'ouverture (112), et
mesure une propriété d'écoulement d'un liquide d'essai (5) dans un tube capillaire (11), le liquide d'essai (5) étant contenu dans la partie réservoir de liquide (4) ayant coulé depuis la seconde partie d'ouverture (112), et
le procédé inclut un premier processus de refoulement dans lequel l'unité de réglage de pression (3) est connectée à la première partie d'ouverture (111) avec une pression positive par rapport à une pression critique, le liquide d'essai (5) ayant coulé dans le tube capillaire (11) est envoyé de la seconde partie d'ouverture (112) à la partie réservoir de liquide (4), et le refoulement est arrêté lorsque le liquide d'essai (5) est refoulé vers l'unité de détection de refoulement (69).

6. Procédé selon la revendication 5, comprenant, après le premier processus de refoulement, un processus de mesure d'une propriété d'écoulement lorsque le liquide d'essai (5) dans la partie réservoir de liquide (4) a été mélangé avec un composant cible d'essai et amené à s'écouler dans le tube capillaire (11).

7. Procédé selon l'une quelconque des revendications 2, 3, 5 ou 6, dans lequel le tube capillaire (11) est incliné.

8. Procédé selon la revendication 7, dans lequel l'unité de réglage de pression (3) peut en outre régler la pression par étapes.

9. Système de mesure d'une propriété d'écoulement d'un liquide d'essai (5), le système inclut un instrument de mesure (101) comprenant :
un moyen de refoulement configuré pour refouler le liquide d'essai (5), le liquide d'essai (5) ayant coulé dans un tube capillaire (11) incluant une première partie d'ouverture (111) et une seconde partie d'ouverture (112) depuis le côté seconde partie d'ouverture (112) vers le côté première partie d'ouverture (111), jusqu'au côté seconde partie d'ouverture (112) ;
une vanne de réglage pour régler le débit de liquide dans le tube capillaire (11) ; et
une unité de mesure (6) configurée pour mesurer un temps d'arrivée tn à une position de niveau de liquide Ln dans le tube capillaire (11), et **(A)** ou **(B):**
**(A)** le moyen de refoulement étant une unité de réglage de pression (3) qui est connectée au côté première partie d'ouverture (111) et peut être fixée à la pression avec laquelle le liquide d'essai (5) ayant coulé dans le tube capillaire (11) est refoulé vers le côté seconde partie d'ouverture (112),
la vanne de réglage incluant une vanne de commutation (2) pour commuter une connexion entre l'unité de réglage de pression (3) et le tube capillaire (11),
l'instrument de mesure (101) incluant une unité de détection configurée pour mesurer une propriété d'écoulement d'un liquide d'essai (5) en utilisant un instrument de mesure (101) incluant une unité de détection de refoulement (69) configurée pour détecter que le liquide d'essai (5) dans le tube capillaire (11) a été refoulé vers le côté seconde partie d'ouverture (112) et l'a traversé, ou
**(B)** le moyen de refoulement incluant un étage incliné pour disposer le tube capillaire (11) et pouvant être installé dans une configuration dans laquelle le côté première partie d'ouverture (111) est positionné plus bas que la seconde partie d'ouverture (112) pour l'écoulement et dans une configuration dans laquelle le côté seconde partie d'ouverture (112) est positionné plus bas que la première partie d'ouverture (111) pour refouler le liquide d'essai (5) dans le tube capillaire (11).

10. Système selon la revendication 9, dans lequel l'instrument de mesure (101) de **(A)**
inclut une partie réservoir de liquide (4) qui vient en contact avec le côté seconde partie d'ouverture (112), et
mesure une propriété d'écoulement d'un liquide d'essai (5) dans un tube capillaire (11), le liquide d'essai (5) étant logé dans la partie réservoir de liquide (4) ayant coulé depuis la seconde partie d'ouverture (112),
l'instrument de mesure (101), incluant un premier moyen de processus de refoulement selon lequel l'unité de réglage de pression (3) est connectée à la première partie d'ouverture (111) avec une pression positive par rapport à une pression critique, le liquide d'essai (5) ayant coulé dans le tube capillaire (11) étant envoyé de la seconde partie d'ouverture (112) à la partie réservoir de liquide (4), et le refoulement étant arrêté lorsque le liquide d'essai (5) est refoulé vers l'unité de détection de refoulement (69), et
l'instrument de mesure (101) incluant, après le premier processus de refoulement, un moyen de mesure d'une propriété d'écoulement lorsque le liquide d'essai (5) dans la partie réservoir de liquide (4) a été mélangé avec un composant cible d'essai et amené à s'écouler dans le tube capillaire (11).
